# EUROPÄISCHE PATENTSCHRIFT

(11) **EP 3 630 018 B1**
(45) Veröffentlichungstag und Bekanntmachung des Hinweises auf die Patenterteilung: **10.05.2023**
(21) Anmeldenummer: 18729931.8
(22) Anmeldetag: 04.06.2018
(51) Int. Cl.: A61F 2/38, A61B 17/17, A61F 2/46

(54) **RADIUSKÖPFCHENIMPLANTAT**
RADIAL-CAPITELLAR IMPLANT
IMPLANT CAPITELLAIRE RADIAL

(30) Priorität: 02.06.2017 DE 102017112244; 13.11.2017 DE 102017126618
(43) Veröffentlichungstag der Anmeldung: 08.04.2020
(73) Patentinhaber: OT Medizintechnik GmbH, 80639 München (DE)
(72) Erfinder: SCHREIBER, Ulrich, 80639 München (DE)
(74) Vertreter: Bobbert & Partner Patentanwälte PartmbB
(86) Internationale Anmeldenummer: PCT/EP2018/064663
(87) Internationale Veröffentlichungsnummer: WO 2018/220233

(56) Entgegenhaltungen:
- EP-A1- 2 561 833
- WO-A1-91/04718
- WO-A1-91/16017
- WO-A2-2005/086939
- WO-A2-2011/017620
- DE-A1- 3 417 923

## Beschreibung

Die Erfindung bezieht sich auf ein Radiusköpfchenimplantat mit einem Kopf hierfür, einem Schaft einem Gewindeanker sowie auf einen Kit, wie in den Ansprüchen definiert. Eine Sägeschablone, eine Unterlegscheibe, ein Kopf-Applikationswerkzeug, ein Schaft-Implantationswerkzeug, ein Knochenbearbeitungswerkzeug, eine Ratsche und ein Verfahren zum Einsetzen des Radiusköpfchenimplantats werden hierin ebenfalls offenbart.

Bei einer traumatischen Fraktur, einer krankhaften Veränderung oder Anomalie eines Radiusköpfchens ist häufig ein synthetischer Ersatz im Sinne eines Radiusköpfchenimplantats erforderlich. An ein derartiges Implantat sind zahlreiche Anforderungen gestellt. Unter anderem ist es wünschenswert, ein Implantat minimalinvasiv einsetzen zu können.

Aus der WO 2011/017620 A2 sind eine ausrichtbare Prothese mit einem System und einem entsprechenden Verfahren bekannt.

In der WO 2005/086939 A2 sind Systeme für den Knochenersatz offenbart.

Eine Gelenkprothese ist aus der EP 2 561 833 A1 bekannt. Dieses Dokument beschreibt ein Radiusköpfchenimplantat mit einem Kopf, einem Gewindeanker und einem Schaft, wobei der Kopf ein Verriegelungselement zum Befestigen des Kopfes aufweist; wobei der Gewindeanker ein selbstschneidendes Gewinde aufweist und wobei der Schaft ein Schaftoberteil und ein Schaftunterteil aufweist, wobei das Schaftunterteil ausgestaltet ist zum Einführen in einen Radius.

In der WO 91/04718 A1 ist eine Fingergelenksprothese offenbart.

Ein System zur Rekonstruktion der distalen Radioulnargelenke (DRUG) sind aus der WO 91/16017 A1 bekannt.

Es ist die Aufgabe der vorliegenden Erfindung, ein Radiusköpfchenimplantat sowie ein Kit anzugeben. Ferner sollen eine Unterlegscheibe für ein Radiusköpfchenimplantat, ein weiteres Radiusköpfchenimplantat, eine Sägeschablone, ein Kopf-Applikationswerkzeug, ein Schaft-Implantationswerkzeug, eine Ratsche, ein Knochenbearbeitungswerkzeug und ein weiteres Verfahren zum Einsetzen eines Radiusköpfchenimplantats offenbart werden.

Die erfindungsgemäße Aufgabe wird gelöst durch das Radiusköpfchenimplantat nach Anspruch 1, sowie durch das Kit nach Anspruch 14. Ferner werden eine Unterlegscheibe für ein Radiusköpfchenimplantat, eine Sägeschablone, ein Kopf-Applikationswerkzeug, ein Schaft-Implantationswerkzeug, eine Ratsche, ein Knochenbearbeitungswerkzeug und ein Verfahren zum Einsetzen eines Radiusköpfchenimplantats offenbart.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Soweit im Folgenden nicht anders ausgeführt, bedeutet proximal zur Körpermitte hin und distal von der Körpermitte weg. In Bezug auf das Radiusköpfchenimplantat ist proximal und distal bezogen auf die Lage des Radiusköpfchenimplantats nach einer bestimmungsgemäßen Implantation zu verstehen.

Der Kopf für ein erfindungsgemäßes Radiusköpfchenimplantat weist ein, insbesondere drehbares, Verriegelungselement zum Befestigen, insbesondere zum lösbaren Befestigen, des Kopfes auf. Vorzugsweise dient das Verriegelungselement dem Befestigen des Kopfes an einem Schaft des Radiusköpfchenimplantats. Das Verriegelungselement ist vorzugsweise ein eigenes Bauteil, das in den Kopf eingesetzt werden kann.

Befestigen des Kopfes am Schaft wie hierin offenbart bedeutet in einigen Ausführungsformen, dass der Kopf mit dem Schaft verbunden ist, wobei jedoch Relativbewegungen in eine(r) oder mehrere(n) Richtungen oder Ebenen gestattet sind, beispielsweise kann eine Rotation des Kopfes um die Längsachse des Schafts weiterhin möglich sein; in anderen Ausführungsformen bedeutet befestigen ein Fixieren, das auch eine Rotation des Kopfes unterbindet.

Der Schaft für ein erfindungsgemäßes Radiusköpfchenimplantat weist ein Schaftoberteil und ein Schaftunterteil auf. Das Schaftoberteil ist dabei zu seiner Befestigung an dem Kopf des Radiusköpfchenimplantats ausgestaltet; mit anderen Worten kann der Kopf am Schaftoberteil befestigt werden. Das Schaftoberteil kann dabei beispielsweise im Wesentlichen die Form eines Tellers oder einer Platte annehmen. Das Schaftunterteil ist zu seinem Einführen in einen Knochen, insbesondere in den Markraum eines Radiusschafts, ausgestaltet. Das Schaftunterteil weist dazu vorzugsweise eine langgestreckte Form auf, die vorzugsweise in Teilen oder im Wesentlichen zylindrisch ist.

Der Gewindeanker für ein erfindungsgemäßes Radiusköpfchenimplantat weist ein - vorzugsweise selbstschneidendes - Gewinde und wenigstens eine, insbesondere zentrale oder dezentrale, Durchgangsöffnung auf.

In einigen Ausführungsformen ist das Gewinde zur Herstellung und Gewährleistung von der Primärstabilität des Radiusköpfchenimplantats ausgestaltet.

In einigen Ausführungsformen ist der Gewindeanker an seinem nach außen gerichteten Teil derart gestaltet, dass eine Osseointegration gefördert wird, beispielsweise durch eine Makro- oder Mikrostrukturierung und/oder eine Beschichtung. Der nach innen gerichtete Anteil (die zentrale Durchgangsöffnung) ist in manchen Ausführungsformen jedoch glatt ausgestaltet (z. B. poliert, beschichtet und/oder gehärtet), damit dieser Teil gegenüber dem Schaft gleiten kann, nur geringe Reibkräfte aufweist, das Abriebverhalten minimiert und/oder die Osseointegration gehemmt ist.

In einigen Ausführungsformen ist der Gewindeanker an seinem nach außen gerichteten Teil mit einer raueren Oberfläche als sein nach innen gerichteter Anteil (die zentrale Durchgangsöffnung) ausgestaltet. Dies kann optional ungeachtet des Gewindes gelten.

Die hierin offenbarte Unterlegscheibe für ein erfindungsgemäßes Radiusköpfchenimplantat ist zum Einsetzen zwischen Schaftoberteil und Knochen, insbesondere zwischen der distalen Oberfläche des Schaftoberteils und Radiusschaft ausgestaltet. Die Unterlegscheibe dient vorzugsweise zur Einstellung des gewünschten Abstandes zwischen dem Radiusschaft (nach Resektion des knöchernen Radiusköpfchens) und der proximalen Seite des Kopfes. Sie dient damit zum Einstellen der Gelenkspannung (zur Vermeidung des Phänomens, das als over-/understuffing bezeichnet wird).

Das erfindungsgemäße Radiusköpfchenimplantat weist einen Kopf (der optional ein Verriegelungselement aufweist), einen Gewindeanker und einen Schaft auf. Diese liegen vorzugsweise jeweils getrennt voneinander vor oder sind voneinander trennbar, insbesondere ohne Werkzeug zu benötigen oder zerstörerisch tätig sein zu müssen.

Das erfindungsgemäße Radiusköpfchenimplantat ist vorzugsweise dazu geeignet, in einer minimalinvasiven Prozedur eingesetzt zu werden. Dazu kann nach einer Resektion des Radiusköpfchens vorzugsweise der Schaft mit aufgesetztem Gewindeanker in den Radius eingeschwenkt werden, was vorteilhafterweise auch in sehr kleinen Resektionshöhlen möglich ist.

Die hierin offenbarte Sägeschablone weist eine Platte mit einer Zunge auf, die ganz oder zumindest abschnittsweise parallel zur Platte angebracht ist, wobei die Zunge vorzugsweise eine Konvexität in Richtung der Platte aufweist. Anstatt der Platte kann die Sägeschablone in einigen Ausführungsformen auch eine für eine oszillierende Säge ausgestaltete Führung aufweisen, beispielsweise einen Schlitz oder eine Öffnung.

Das hierin offenbarte Kopf-Applikationswerkzeug ist ausgestaltet und vorgesehen zum Aufnehmen des Kopfes des erfindungsgemäßen Radiusköpfchenimplantats und zum Befestigen des Kopfes auf einem in einen Radius eingesetzten Schaft.

Das hierin offenbarte Schaft-Implantationswerkzeug ist insbesondere ausgestaltet und vorgesehen zum Einsetzen des Schafts mit Gewindeanker oder anderem Anker in den Markraum eines Radius. In einigen Ausführungsformen kann das Schaft-Implantationswerkzeug auch verwendet werden, um den Schaft ohne einen Anker, insbesondere den Gewindeanker, in den Knochen einzusetzen, insbesondere wenn der Schaft mit Knochenzement und ohne Anker eingesetzt wird.

Das hierin offenbarte Knochenbearbeitungswerkzeug weist einen Stiel und ein am Stiel seitlich angeordnetes Raspelelement auf.

Die hierin offenbarte Ratsche ist zum Eindrehen eines Gewindeankers in einen Radius mittels einer Rotation des Schafts ausgestaltet.

Vorzugsweise kann die Ratsche auch zur Explantation mit einem umgekehrten Freilauf verwendet werden. Alternativ kann in einigen Fällen die Ratsche zur Explantation umgekehrt, z. B. umgedreht, verwendet werden, wobei der Freilauf nicht umgekehrt wird.

Das erfindungsgemäße Kit umfasst das erfindungsgemäße Radiusköpfchenimplantat und mindestens eines der folgenden, hierin offenbarten Hilfsmittel:
- Sägeschablone;
- Kopf-Applikationswerkzeug;
- Schaft-Implantationswerkzeug;
- Ratsche; und/oder
- Knochenbearbeitungswerkzeug.

Das hierin offenbarte Verfahren zum Einsetzen eines Radiusköpfchenimplantats umfasst mindestens eine Vielzahl (d. h. zwei oder mehr) der folgenden Schritte:
- Anlegen und/oder Ausrichten einer Sägeschablone an ein/einem Radiusköpfchen, ein/einem Capitulum humeri und/oder dessen Gelenkspalt und Anlegen und/oder Ausrichten der Sägeschablone entlang eines Radiusschafts;
- Größenbestimmung des Radiusschafts und/oder Radiusköpfchens;
- Resektion des Radiusköpfchens entlang einer vom Radiusköpfchen distal gelegenen Seite einer Platte oder Führung der vorzugsweise hierin offenbarten Sägeschablone;
- Erweitern des Markraums des Radius durch ein vorzugsweise wie hierin offenbartes Knochenbearbeitungswerkzeug;
- Einführen eines vorzugsweise wie hierin offenbarten Schafts mit Gewindeanker in den Markraum des Radius mit einem Schaft-Implantationswerkzeug;
- Eindrehen des vorzugsweise wie hierin offenbarten Schafts mit Gewindeanker in den Radius mit einer Ratsche;
- Aufsetzen eines Kopfes, vorzugsweise eines erfindungsgemäßen Radiusköpfchenimplantats, auf den Schaft mit einem Kopf-Applikationswerkzeug;
- Verriegeln eines Verriegelungselements des vorzugsweise wie hierin offenbarten Kopfs durch das Kopf-Applikationswerkzeug; und
- optional: Einsetzen einer Unterlegscheibe, vorzugsweise wie hierin offenbart.

Vorteilhafte Weiterentwicklungen der vorliegenden Erfindung sind jeweils Gegenstand von Unteransprüchen und Ausführungsformen.

Wenn hierin von einer Ausführungsform die Rede ist, so ist damit eine exemplarische, erfindungsgemäße Ausführungsform zu verstehen, die nicht als beschränkend zu verstehen ist.

Erfindungsgemäße Ausführungsformen können eines oder mehrere der oben und/oder im Folgenden genannten Merkmale in jeder beliebigen Kombination aufweisen, sofern eine solche für den Fachmann nicht als technisch unmöglich zu erkennen ist.

Das Verriegelungselement ist vorzugsweise drehbar ausgeführt. Alternativ kann das Verriegelungselement auch als Einführkaskade, die optional auch verschlossen werden kann, ausgeführt sein.

Das optional drehbare Verriegelungselement des Kopfes ist vorzugsweise zum Befestigen des Kopfes am Schaft ausgestaltet. Dazu weist das Verriegelungselement in einigen Ausführungsformen einen Riegel auf, mit dem ein Teil des Schafts im Kopf verriegelt werden kann. Das Verriegelungselement kann in einer Öffnung, insbesondere in einem Sackloch, des Kopfes eingesetzt sein. Vorzugsweise ist das Verriegelungselement derart ausgestaltet, dass seine nach außen gerichtete Oberfläche in einer geschlossenen Rotationsstellung bündig mit der Kopfkontur abschließt.

In einigen Ausführungsformen weist der Riegel optional die Form eines Schlüsselbarts auf. Das optional drehbare Verriegelungselement kann den Kopf am Schaft verriegeln, indem sich der Riegel mittels einer Drehung des Verriegelungselements optional formschlüssig am Schaft einhakt. Dadurch kann das Verriegelungselement einerseits im Kopf gegen ein Herausziehen aus dem Kopf gesichert und andererseits der Kopf am Schaft, vorzugsweise formschlüssig, fixiert werden.

Das Verriegelungselement ist vorzugsweise derart ausgestaltet, dass sich durch ein Drehen des Verriegelungselements ein lösbares Befestigen und/oder Verriegeln des Kopfes am Schaft ergeben kann.

Das Verriegelungselement weist vorzugsweise eine Verliersicherung auf, die derart mit dem Kopf zusammenwirkt, dass das Verriegelungselement im Kopf eingerastet oder eingeklemmt wird.

In einigen Ausführungsformen ist die Verliersicherung kein kraftschlüssiges Sicherungselement, keine Klemmschraube, keine Fixierschraube und/oder kein Sicherungsstift.

In einigen Ausführungsformen weist das Verriegelungselement kein T-Profil auf. Ein T-Profil kann zum formschlüssigen Verbinden des Schafts mit dem Kopf ausgestaltet sein.

In einigen Ausführungsformen weist das Verriegelungselement keinen Pin oder keinen Befestigungsstift auf. Der Pin bzw. der Befestigungsstift könnte zum formschlüssigen Sichern des Kopfes auf oder an dem Schaft ausgestaltet sein.

In einigen Ausführungsformen weist das drehbare Verriegelungselement mindestens ein Rastelement für mindestens eine Rotationsstellung auf.

Das Rastelement kann dabei in einigen Fällen dazu dienen, das Verriegelungselement in einer oder mehreren Rotationsstellung(en) einrasten zu lassen. Dazu ist der Kopf vorzugsweise ausgestaltet, um mit dem Rastelement des Verriegelungselements zusammenzuwirken. Beispielsweise kann das Verriegelungselement als Rastelement mindestens eine Rastnase aufweisen, die in mindestens einer Rastvertiefung im Kopf, insbesondere in der Öffnung des Kopfes, einrasten kann.

In einigen Ausführungsformen kann das Verriegelungselement in einer offenen Rotationsstellung, in der der Schaft vom Kopf gelöst werden kann, und/oder in einer geschlossenen Rotationsstellung, in der der Kopf am Schaft befestigt ist, einrasten.

In einigen Ausführungsformen weist das Verriegelungselement einen Antrieb auf. Ein Antrieb ist eine Einrichtung, an der ein Werkzeug ansetzen kann und ein Drehmoment auf das Verriegelungselement übertragen kann, um das Verriegelungselement zu drehen oder anderweitig zu betätigen. Auf diese Weise kann beispielsweise das Verriegelungselement von einer offenen zu einer geschlossenen Stellung gedreht (oder bewegt) werden oder umgekehrt.

Das Verriegelungselement kann vorzugsweise durch eine Rotation um etwa 90° von einer offenen zu einer geschlossenen Rotationsstellung überführt werden und umgekehrt.

In einigen Ausführungsformen umfasst ein Antrieb einen Innensechskant und/oder einen Schlitz, in den jeweils ein Drehwerkzeug eingreifen kann.

In einigen Ausführungsformen ist der Kopf derart gestaltet, dass er in Funktionsstellung in Kontakt mit dem entsprechenden Capitulum humeri oder dem Ersatz eines Capitulum humeri stehen kann. Dabei weist in einigen Ausführungsformen der Kopf eine glatte oder sehr glatte Oberfläche und/oder keine scharfen Kanten auf. Zudem oder alternativ ist das Material des Kopfes vorzugsweise derart ausgestaltet, dass sich eine Hart-Weich-Paarung zwischen Kopf und Capitulum humeri ergibt. Alternativ kann das Material des Kopfes derart ausgestaltet sein, dass sich eine Hart-Hart-Paarung ergibt, beispielsweise durch Keramikoberflächen.

In einigen Ausführungsformen weist der Kopf auf der in Funktionsstellung dem Capitulum humeri zugewandten Seite eine konkave Krümmung oder Tellerkrümmung auf.

In einigen Ausführungsformen werden zum Einsatz des Radiusköpfchenimplantats mehrere, vorzugsweise fünf, unterschiedliche Kopfgrößen bereitgestellt. Dabei weisen die unterschiedlichen Kopfgrößen vorzugsweise eine proportionale Tellerkrümmung und -tiefe auf. Dabei ist die äußere Kontur des Kopfes angepasst für eine vorteilhafte radio-ulnare Gelenkwirkung. Vorzugsweise sind die konkave Krümmung und der Durchmesser des Kopfes für eine vorteilhafte radiocapituläre Artikulation und/oder radio-ulnare Artikulation angepasst. Zudem ist vorzugsweise der Kopf derart ausgestaltet, dass die Artikulation zwischen Kopf und medialer Seite der Trochlea vorteilhaft zu einer Stabilisierung führt.

In einigen Ausführungsformen werden auch der Schaft und/oder der Gewindeanker in mehreren Größen, insbesondere in fünf Größen, bereitgestellt. Dabei können vorzugsweise alle Schaftgrößen mit allen Kopfgrößen kombiniert werden.

In einigen Ausführungsformen kann der Kopf derart am Schaft befestigt und/oder verriegelt werden, dass eine Bewegung des Kopfes möglich ist, insbesondere eine Relativbewegung zwischen Kopf und Schaft, insbesondere eine Rotation um die Längsachse des Schafts, was eine Kompensation einer eventuell vorhandenen Inkongruenz zwischen Artikulationsfläche des Kopfes und Capitulum humeri ausgleichen kann. Bei freier Rotation des Kopfes kann die translatorische Bewegung des Kopfes senkrecht zur Längsachse des Schaftunterteils und/oder entlang dieser Längsachse dabei frei sein oder behindert werden.

In anderen Ausführungsformen kann der Kopf derart am Schaft befestigt werden, dass eine Bewegung, insbesondere eine Rotation, des Kopfes unterbunden wird. Bei unterbundener Rotation des Kopfes kann die translatorische Bewegung des Kopfes senkrecht zur Längsachse des Schaftunterteils und/oder entlang dieser Längsachse dabei frei sein oder behindert werden.

In einigen Ausführungsformen ist die proximale Kontur des Kopfes rotationssymmetrisch und weist eine konkave Oberfläche auf. Dabei entspricht der Radius dieser konkaven Kontur in einigen Fällen nicht in jeder Winkelstellung des Kopfes zum Capitulum humeri der konvexen Krümmung des Capitulum humeri. Zudem kann sich in einigen Fällen das Maximum des Capitulum humeri gegenüber dem Minimum des Kopfes im Rahmen einer Bewegung verschieben oder wandern.

In einigen Ausführungsformen weist der Kopf an seiner Unterseite, die in Gebrauchsstellung oder Funktionsstellung vom Capitulum humeri abgewandt ist, eine Öffnung auf. Diese Öffnung besitzt in einer Erstreckungsrichtung mindestens zwei unterschiedliche Abmessungen. Vorzugsweise bestehen in einer Erstreckungsrichtung quer zur Haupterstreckungsrichtung der Öffnung mindestens zwei verschiedene Abmessungen der Öffnung. In einigen Ausführungsformen befindet sich die Öffnung seitlich am Kopf.

In einigen Ausführungsformen weist die Öffnung an der Unterseite des Kopfes einen schmalen Anteil und einen weiteren Anteil auf. In einigen Ausführungsformen weist die Öffnung die Kontur eines Schlüsselloches auf. Die Kontur eines Schlüssellochs besteht in einigen Fällen aus einem (mehr oder weniger vollständigen) Kreis, an den sich ein Rechteck mit seiner kürzeren Seite unmittelbar anschließt. Dabei ist der (ggf. fiktive) Durchmesser des Kreises größer als die kürzere Seite des Rechtecks misst.

In einigen Ausführungsformen umfasst der Gewindeanker für ein Radiusköpfchenimplantat ein selbstschneidendes Gewinde zum Eindrehen in den kortikalen und/oder spongiösen Knochen. Zudem umfasst der Gewindeanker vorzugsweise eine zentrale Durchgangsöffnung zum Einschieben eines Schaftunterteils. Der Schaft ist vorzugsweise innerhalb des Gewindeankers beweglich, insbesondere in axialer Richtung des Schaftunterteils. Vorzugsweise kann der Schaft nicht relativ zum Gewindeanker rotiert werden.

Der Gewindeanker weist vorzugsweise ein Trapezgewinde auf, das besonders vorzugsweise selbsthemmend ausgestaltet ist, um einem Lösen entgegenzuwirken.

Das Gewinde des Gewindeankers ist in einigen Ausführungsformen auch bei Linksdrehung selbstschneidend. Dadurch kann in einigen Fällen eine Explantation erleichtert werden.

Einzelne Freiheitsgrade können an den Schnittstellen zwischen den Komponenten (Kopf, Gewindeanker und/oder Schaft) in definiertem Umfang gezielt frei bleiben, um z.B. ein physiologisches Einstellen zu den Gelenkflächen von Radius, Ulna, Capitulum humeri und/oder Trochlea zu ermöglichen. In einigen Ausführungsformen besitzt das Radiusköpfchenimplantat zwei Freiheitsgrade: der Kopf ist um die z-Achse (Längsachse des Schaftunterteils) drehbar und der Schaft ist entlang der z-Achse und/oder quer zur z-Achse beweglich. In anderen Ausführungsformen besitzt das Radiusköpfchenimplantat nur einen der beiden genannten Freiheitsgrade. In einigen Ausführungsformen besteht kein Freiheitsgrad: Kopf und Schaft sind fest. In einigen Ausführungsformen ist der Kopf entlang der x-Achse und der y-Achse um +/- 1 mm beweglich, so dass zwei Freiheitsgrade bestehen.

In einigen Ausführungsformen erlaubt der Gewindeanker eine Fixierung am Isthmus des Markraums.

Der Gewindeanker verjüngt sich in einigen Ausführungsformen nach distal, das heißt in Richtung des Endes, das zuerst in den Knochen eingeführt werden soll. In einigen Ausführungsformen stellt die Grundform des Gewindeankers einen Kegelstumpf dar, in den koaxial eine Durchgangsöffnung eingebracht ist und auf dessen Mantelfläche ein selbstschneidendes Gewinde aufgebracht ist. Das Gewinde ist dabei vorzugsweise periodisch unterbrochen, beispielsweise zwei-, drei- oder viermal entlang des kreisförmigen Umfangs, um das abgetragene Knochenmaterial abzutransportieren.

In einigen Ausführungsformen weist die Durchgangsöffnung des Gewindeankers mindestens ein Übertragungselement auf zum Übertragen eines Drehmoments von einem eingeführten Schaftunterteil auf den Gewindeanker. Ein solches Übertragungselement umfasst dabei vorzugsweise ein, zwei oder mehrere Rinnen, Kerben, Vorsprünge, Zähne, Protrusionen, Stege, Nasen oder Grate. Der Schaft weist vorzugsweise Führungseinrichtungen auf, die mit den Übertragungselementen des Gewindeankers zusammenwirken, um ein Drehmoment vom Schaftunterteil auf den Gewindeanker zu übertragen. Dazu weist das Schaftunterteil beispielsweise eine oder mehrere Rinne(n) auf, in die die Nase(n) des Gewindeankers eingreifen können, um ein Drehmoment vom Schaft auf den Gewindeanker zu übertragen. Zugleich kann oder können vorzugsweise das oder die Übertragungselemente des Gewindeankers mit der beziehungsweise den Führungseinrichtung(en) eine Linearführung darstellen, so dass der Schaft entlang der Längsachse des Schaftunterteils im Gewindeanker beweglich bleibt, nachdem der Schaft mit dem Gewindeanker in den Radius eingeschraubt wurde.

Der Schaft für ein erfindungsgemäßes Radiusköpfchenimplantat weist ein Schaftunterteil auf, das in einigen Ausführungsformen dazu ausgestaltet ist, in die Durchgangsöffnung des Gewindeankers eingeführt zu werden. Das Schaftunterteil ist dabei vorzugsweise mit Führungseinrichtungen versehen, die mit Übertragungselementen des Gewindeankers zusammenwirken, so dass ein Drehmoment von dem Schaft auf den Gewindeanker übertragen werden kann.

In einigen Ausführungsformen weisen der Gewindeanker und/oder der Schaft Elemente zum lösbaren Fixieren des Gewindeankers am Schaft auf, so dass sich vorzugsweise eine Verliersicherung des Schafts gegenüber dem Gewindeanker ergibt. Dabei kann der Gewindeanker in einigen Fällen am Schaft festgeklemmt und/oder dort verrastet werden. Beispielsweise kann dazu am Übergang zwischen Schaftunterteil und Schaftoberteil das Schaftunterteil einen größeren Durchmesser annehmen, so dass der Gewindeanker dort festgeklemmt und/oder verrastet werden kann. So wird ein Abrutschen des Gewindeankers vom Schaft während der operativen Implantation des Radiusköpfchenimplantats vermieden. In einigen Fällen wird der Schaft mit einem bereits verliersicher aufgesetzten Gewindeanker bereitgestellt.

In einigen Ausführungsformen weist der Schaft am Übergang vom Schaftunterteil zum Schaftoberteil wenigstens eine, optional umlaufende, Nut auf. Die Nut kann beispielsweise Teil der Verliersicherung sein, wobei in die Nut beispielsweise Anteile des Gewindeankers einrasten können. Die Nut kann alternativ oder zusätzlich dazu dienen, Unterlegscheiben am Schaft zu befestigen.

Anstelle einer Nut kann/können eine oder mehrere Erhebung(en) am Schaft vorgesehen sein, die ihrerseits in eine oder mehrere Nut(en) des Gewindeankers einrasten können.

In einigen Ausführungsformen weist das Schaftoberteil einen Haltestift auf. Der Haltestift ist vorzugsweise an der dem Kopf zugewandten Seite des Schafts angebracht. Der Haltestift hat dabei vorzugsweise an seinem dem Schaftoberteil zugewandten Abschnitt einen kleineren Querschnitt als an einem weiter vom Schaftoberteil entfernt angeordneten Abschnitt. In einigen Ausführungsformen nimmt der Haltestift die Form eines Pilzkopfes an.

Vorzugsweise liegt das Schaftoberteil nach der Implantation bündig auf dem Radiusschaft bei reseziertem Radiusköpfchen auf. Dabei kann das Schaftoberteil auf der zum Radiusknochen hin gerichteten Seite vorzugsweise eine osseointegrative Fläche aufweisen. Die osseointegrative Fläche ist dabei vorzugsweise rau und/oder weist eine Makrostruktur und/oder Beschichtung auf. Die andere, nach proximal gerichtete Seite des Schaftoberteils hat vorzugsweise eine glatte, insbesondere gehärtete und/oder polierte und/oder beschichtete Oberfläche, die vorzugsweise in der Artikulation mit dem Radiuskopf wenig Reibung und/oder Abrieb erzeugt und/oder durch eine formschlüssige Verbindung zwischen Kopf und Schaftoberteil eine Relativbewegung einschränkt oder verhindert.

Aufgrund eines beim bestimmungsgemäßen Gebrauch bündig aufliegenden Schaftoberteils ist die primäre Ausrichtung des Schafts und Kopfs vorzugsweise durch die Resektionsausrichtung bestimmt.

In einigen Ausführungsformen kann der Haltestift in die Öffnung an der Unterseite des Kopfes eingeführt werden. Dabei kann ein breiterer Anteil des Haltestifts vorzugsweise in einen breiteren Anteil der Öffnung des Kopfes eingeführt werden, jedoch vorzugsweise nicht in einen schmaleren Anteil der Öffnung. Durch Verschieben des Haltstifts nach dem Einsetzen in die Öffnung hin zu dem schmaleren Anteil der Öffnung kann der Haltestift axial vorzugsweise nicht mehr aus der Öffnung entfernt werden. In einigen Ausführungsformen ist der Haltestift ein Pilzkopf und die Öffnung hat eine Schlüssellochform, dabei kann der Pilzkopf durch den runden Anteil des Schlüssellochs eingeführt werden und danach zum schmaleren Anteil des Schlüssellochs verschoben werden, wobei man sich den schmaleren Stiel des Pilzkopfs zunutze macht; nach dem Verschieben kann der Pilzkopf vorzugsweise axial nicht mehr aus dem Schlüsselloch entfernt werden.

In einigen Ausführungsformen ist das Verriegelungselement des Kopfes dazu ausgestaltet, es in einer offenen Position zu ermöglichen, dass der Haltestift in die Öffnung des Kopfes eingeführt wird und dass in einer geschlossenen Position verhindert wird, dass der Haltestift vom Kopf entfernt wird. In einigen Ausführungsformen wird dies dadurch realisiert, dass das Verriegelungselement ein Verschieben des in die Öffnung eingesetzten Haltestifts entlang der Öffnung verhindert, beispielsweise durch einen Riegel des Verriegelungselements. Durch den Riegel kann beispielsweise ein Formschluss zwischen Kopf (insbesondere dem Verriegelungselement) und Haltestift entstehen.

In einigen Ausführungsformen kann der Schaft, der in jeder Ausführungsform zylindrisch oder konisch oder auf andere Weise zulaufend ausgestaltet sein kann, auch ohne Gewindeanker in den Markraum eingebracht werden und dort mittels Knochenzement verankert werden.

Der Schaft dient bei der Implantation dazu, in den Markraum des Radius vorgeschoben zu werden. Dabei kann in einigen Ausführungsformen auch nach dem Einschrauben des Gewindeankers in den Radius eine Relativbewegung zwischen Gewindeanker und Schaft vorübergehend oder dauerhaft möglich sein.

Das Schaftoberteil weist, gemessen von einer Längsachse des Schaftunterteils, eine Erstreckung in eine erste Richtung auf, die sich unterscheidet von einer Erstreckung in eine zweite Richtung. Das Schaftoberteil kann beispielsweise eine Begrenzung aufweisen, die einen Kreisbogen darstellt dessen Enden mit einer geraden Linie verbunden sind. In einigen Ausführungsformen ist das Schaftoberteil besonders flach ausgestaltet und/oder weist eine abgerundete Kontur auf, was das Einschwenken des Schafts in den Radius vor allem in einer minimalinvasiven Prozedur erleichtern kann.

Vorzugsweise weist das Material des Schafts mindestens eine der folgenden Eigenschaften auf: ausreichende Duktilität, minimaler Abrieb, harte Kontaktflächen, glatte Kontaktflächen.

In einigen Ausführungsformen des Kopfes, des Schafts, des Gewindeankers und/oder der Unterlegscheibe ist diese(r) jeweils aufgrund seines/ihres Materials oder einer zusätzlichen Beschichtung antibakteriell und/oder weist einen strukturierten und/oder beschichteten, osseointegrativen Oberflächenbereich auf.

Die Sägeschablone dient der präzisen Resektion des Radiusköpfchens. Die Sägeschablone ist vorzugsweise in mehreren Größen verfügbar. Die Sägeschablone weist eine Platte, insbesondere eine Sägeplatte, auf, insbesondere zur Führung einer oszillierenden Säge (insbesondere mit einem oszillierenden Sägeblatt mit stirnseitiger Schnittfläche). Statt der Platte kann die Sägeschablone auch mit einer anders gestalteten Führung, insbesondere einer Öffnung zur Führung einer oszillierenden Säge, ausgestattet sein. Dabei hat die Platte vorzugsweise eine Kontur, um die Platte an den Radius, insbesondere an den Radiusschaft, anzulegen. Dazu weist die Platte vorzugsweise einen Bogen auf, der sich am Radiusköpfchen anlegen kann. Durch den Bogen kann der Radiusschaft beziehungsweise das Radiusköpfchen großflächig kontaktiert werden, was die Führung der Säge und deren Stabilisierung beim Schneidvorgang verbessert und als Größenbestimmung fungieren kann.

Zudem weist die Sägeschablone eine Zunge auf, die zusätzlich oder alternativ an das Capitulum humeri angelegt werden kann. In einigen Ausführungsformen folgt die Kontur der Platte in Teilen einem Kreisbogen. Parallel zur Platte ist vorzugsweise über ein Verbindungselement die Zunge angebracht. Das Verbindungselement ist vorzugsweise rechtwinklig zu Zunge und Platte angebracht. Die Zunge weist vorzugsweise in einem Anteil eine Konvexität (insbesondere in Löffelform) in Richtung der Platte auf, das heißt, dass die Zunge in die Richtung der Platte vorgewölbt ist. Die Form der Zunge kann dabei der Größenzuordnung und/oder Ausrichtung der Sägeschablone dienen. Die Sägeschablone kann derart auf einen Radiusknochen aufgesetzt werden, dass der Bogen der Platte seitlich am Radiusköpfchen oder am Radiusschaft anliegt und die Zunge sich im Gelenkspalt zwischen Radiusköpfchen und Humerus befindet, wobei die Zunge mit ihrer Konvexität vorzugsweise der konkaven Oberfläche des Radiusköpfchens anliegt. Das Verbindungselement ist vorzugsweise mit mindestens einem Bohrloch oder einer Durchgangsöffnung versehen, das/die vorzugsweise nicht im rechten Winkel zu dem Verbindungselement verläuft. Hier hindurch können vorzugsweise Drähte (insbesondere K-Draht) zur Fixierung der Sägeschablone, beispielsweise am Humerus, geschoben werden.

In einer Ausführungsform sind an der Sägeschablone, die sowohl links als auch rechts verwendet werden kann, insbesondere dem Verbindungselement, mindestens zwei Öffnungen angebracht, vorzugsweise eine für die Verwendung links und eine weitere für eine Verwendung am rechten Arm. Die Öffnungen sind dabei vorzugsweise entsprechend ihrer Verwendung gekennzeichnet, beispielsweise mit R und L.

Die Sägeschablone kann auch in einigen Ausführungsformen dazu dienen, die Größe des zu ersetzenden Radiusköpfchens zu bestimmen. Dazu liegen vorzugsweise in einem Kit mehrere Sägeschablonen in unterschiedlichen Größen vor, die sich beispielsweise in dem Radius des Bogens der Platte und/oder der Größe der Zunge (insbesondere ihrer Konvexität) unterscheiden können. Zusätzlich oder alternativ können sich die Sägeschablonen in ihrer Ausdehnung in Längsrichtung unterscheiden, um beispielsweise die Tiefe der Trochlea aufzuzeigen.

Nach dem Aufsetzen der passenden Sägeschablone kann entlang der distalen Seite der Platte (bezogen auf das Radiusköpfchen) eine oszillierende Säge geführt werden, so dass der Radius bzw. der Radiuskopf in einer definierten Ebene reseziert werden kann. Dazu kann ein Teil der Platte als Griff zum Handling der Platte verwendet werden.

In einigen Ausführungsformen kann die Sägeschablone in Zusammenhang mit einer Größenlehre verwendet werden. Die Platte kann dabei eine Führung für einen Schlitten der Größenlehre aufweisen. Bei einer in den Gelenkspalt eingeführten Zunge kann die Größenlehre entlang der Führung an das Radiusköpfchen herangeschoben werden, so dass das Radiusköpfchen seitlich einen Arm der Größenlehre berührt. Der Arm wird so durch das Radiusköpfchen ausgelenkt, dass ein mit dem Arm verbundener Zeiger auf einer Skala die Größe des Radiusköpfchens anzeigt. Die Teile des erfindungsgemäßen Radiusköpfchenimplantats sowie die zugehörigen Werkzeuge können anhand der von der Skala abgelesenen Werte ausgewählt werden.

Dadurch kann in einigen Fällen vorteilhafterweise eine Resektion durchgeführt werden, die eine für eine nachfolgende Implantation eines Radiusköpfchenimplantats optimale Voraussetzung erzeugt.

Eine Schablone, die mehrere kreisförmige Vertiefungen aufweist und die einem Kit beiliegen kann, kann verwendet werden, um die Größe des Radiusköpfchens nach der Entnahme zu überprüfen. Dazu wird das resezierte Radiusköpfchen in die Vertiefungen eingelegt und überprüft, ob es mit den Maßen der Vertiefung übereinstimmt. Die Vertiefungen sind vorzugsweise gekennzeichnet, um daraufhin das passende Radiusköpfchenimplantat auszuwählen.

Das hierin offenbarte Knochenbearbeitungswerkzeug weist vorzugsweise einen flachen Stiel auf, an dem zu einem Ende hin auf einer Seite ein Raspelelement angebracht ist. Das Raspelelement läuft vorzugsweise rechtwinklig vom Stiel weg konisch zu. In einigen Ausführungsformen hat das Raspelelement im Wesentlichen die Form eines Kegelstumpfs. Die Oberfläche des Raspelelements ist vorzugsweise wie die Oberfläche einer Raspel oder Feile ausgestaltet, weist also beispielsweise Einkerbungen oder Zähne auf.

Nach der Resektion des Radiusköpfchens kann das Knochenbearbeitungswerkzeug mit dem Raspelelement in den Markraum des Radius eingeführt werden. Durch Drehen des Knochenbearbeitungswerkzeugs kann der Markraum spanend erweitert werden. Das Raspelelement weist vorzugsweise eine Bauhöhe auf, die eine minimalinvasive Einführung in den Bearbeitungsraum ermöglicht. In einigen Ausführungsformen kann das Knochenbearbeitungswerkzeug eine Ratschenfunktion haben.

Nach einer ausreichenden Vergrößerung des Markraums durch das Knochenbearbeitungswerkzeug kann der Schaft mit aufgesetztem Gewindeanker in den Radius eingeführt werden. Ausreichend bedeutet hier, dass der Durchmesser des geweiteten Markraums vorzugsweise dem Kerndurchmesser des Gewindeankers entspricht.

Die Form der Raspel und der Vorgang des Aufweitens des Knochenlagers (des Radiusschafts) ist vorzugsweise derart gestaltet, dass der Schaft nicht gesprengt und das Knochenlager mechanisch nicht übermäßig belastet wird. Auch die anschließende Implantation des Schafts und das Eindrehen des Gewindeankers geschehen vorzugsweise so, dass das Knochenlager nicht übermäßig belastet wird.

In einigen Ausführungsformen kann das Knochenbearbeitungswerkzeug zweiteilig ausgeführt sein. Ein erster Teil umfasst dabei vorzugsweise einen Stiel, einen Handgriff und/oder eine Ratsche zur Manipulation (insbesondere für eine Dreh- oder Schwenkbewegung um die z-Achse des Radiusschafts) des, insbesondere auswechselbaren, zweiten Teils, insbesondere eines Raspelelements. Die Größe der Raspel kann dabei vorzugsweise von klein nach groß schrittweise verwendet werden. Dabei ist das Raspelelement in z-Richtung des Stiels verfahrbar. Dies kann es ermöglichen, dass der Stiel immer flächig auf der Resektionsfläche des Radiusschaftes aufliegt und so die Raspelrichtung senkrecht dazu vorgegeben ist.

Zum Einführen des Schafts mit aufgesetztem Gewindeanker in den Radius kann das Schaft-Implantationswerkzeug verwendet werden. Das Schaft-Implantationswerkzeug umfasst vorzugsweise den Haltestift des Schaftoberteils, insbesondere an der schmaleren Stelle des Haltestifts. Zudem kann in einigen Ausführungsformen das Schaft-Implantationswerkzeug mit einem oder mehreren Stift(en) in ein oder mehrere Loch/Löcher des Schaftoberteils eingreifen. Das Schaft-Implantationswerkzeug weist vorzugsweise ein elastisches Element auf, insbesondere eine Feder, um den Haltestift am Schaft-Implantationswerkzeug zu fixieren. In einigen Ausführungsformen ist oder umfasst das Schaft-Implantationswerkzeug eine Zange, deren Zangenfläche derart geformt ist, dass sie den Haltestift des Stiels umfassen kann.

In einigen Fällen kann der Schaft mit aufgesetztem Gewindeanker wie folgt eingeführt werden: Das Schaftoberteil wird zunächst mit seiner distalen Seite auf die Resektionsfläche des Radiusschafts aufgesetzt. Dies kann zur Folge haben, dass die Ausrichtung des Implantats bei und nach dem Einschrauben anatomisch korrekt ist und der Resektionsfläche (xy-Ebene) entspricht. Erst durch ein Herunterdrücken von Stiften des Schaft-Implantationswerkzeugs, das heißt durch das Lösen einer Verliersicherung des Schaft-Implantationswerkzeugs, auf den Gewindeanker greift der Gewindeanker im Knochen. Eine darauffolgende Drehbewegung (in Gewinderichtung) und die vorzugsweise selbstschneidende Form des Gewindeankers verursachen das Einschneiden nach distal und führen somit zur Verankerung des Gewindeankers im Knochen (sogenannte Primärstabilität).

Nach dem Einführen des Schafts mit Gewindeanker durch das Schaft-Implantationswerkzeug kann der Gewindeanker in den Radius eingeschraubt werden. Dazu kann die Ratsche verwendet werden. Die Ratsche weist vorzugsweise Elemente auf, die ein Drehmoment auf Elemente des Schaftoberteils übertragen können. Vorzugsweise weist das Schaftoberteil eine oder mehrere Öffnung(en) auf, in die ein oder mehrere Stifte der Ratsche eingreifen können. In einer Ausführungsform weist die Ratsche wenigstens oder genau jeweils einen, zwei oder mehr Stift(e) und/oder das Schaftoberteil wenigstens oder genau jeweils eine, zwei oder mehr Öffnung(en) auf. Durch Betätigen der Ratsche kann so ein Drehmoment von Ratsche auf Schaft und Gewindeanker übertragen werden, so dass der Gewindeanker in den Radius eingeschraubt wird. Vorzugsweise weist die Ratsche eine Aufnahme für den Haltestift des Schafts auf. In einigen Ausführungsformen sind die wenigstens ein, zwei oder mehr Stifte und/oder die Aufnahme der Ratsche an einem Aufsatz der Ratsche vorhanden. Der Aufsatz kann dabei optional auswechselbar sein und insbesondere in verschiedenen Größen vorliegen. Die Größen des Aufsatzes sind vorzugsweise an verschiedene Schaftgrößen angepasst.

In einigen Ausführungsformen kann das Einsetzen des Stifts/der Stifte der Ratsche in den Schaft dazu führen, dass sich die Verliersicherung des Gewindeankers löst.

Ein Stiel der Ratsche weist vorzugsweise eine Breite von weniger als 15 mm, besonders vorzugsweise weniger als 12 mm, besonders vorzugsweise weniger als 10 mm und besonders vorzugsweise weniger als 9 mm auf. Vorzugsweise ist der Stiel der Ratsche mindestens 5 mm, besonders vorzugsweise mindestens 7 mm und ganz besonders vorzugsweise mindestens 8 mm breit.

Vorzugsweise ist die Dicke des Stiels der Ratsche weniger als 6 mm, besonders vorzugsweise weniger als 5 mm, besonders vorzugsweise weniger als 4 mm und ganz besonders vorzugsweise weniger als 3 mm. Dabei beträgt die Dicke des Stiels der Ratsche vorzugsweise mindestens 1,5 mm, besonders vorzugsweise mindestens 2 mm.

Das Verhältnis von Breite zu Dicke des Stiels der Ratsche ist vorzugsweise mindestens 4, besonders vorzugsweise mindestens 6, besonders vorzugsweise mindestens 8 und ganz besonders vorzugsweise mindestens 9. Dabei ist das genannte Verhältnis vorzugsweise weniger als 15, besonders vorzugsweise weniger als 12 und ganz besonders vorzugsweise weniger als 10.

Die genannten Maße für Dicke und Breite des Stiels der Ratsche gelten vorzugsweise in einem Bereich zwischen 1 cm und 8 cm von der Stelle, an der die Ratsche auf den Schaft aufgesetzt wird, insbesondere von dort gemessen, wo der Haltestift in die Ratsche oder deren Aufsatz eingreift. Besonders vorzugsweise befindet sich dieser Bereich zwischen 2 und 6 cm, ganz besonders vorzugsweise zwischen 3 und 5 cm der genannten Stelle.

In einigen Ausführungsformen bleibt der Schaft nach dem Einschrauben in den Radius axial (bezogen auf die Längsachse des Schaftunterteils) im Gewindeanker beweglich, insbesondere axial verschiebbar.

Daraufhin kann der Kopf mit einem Kopf-Applikationswerkzeug auf den Schaft aufgesetzt werden. Dabei wird vorzugsweise der Haltestift des Schaftoberteils in die Öffnung des Kopfes eingeführt. Durch eine Drehung des Kopf-Applikationswerkzeugs, das zu einer Drehung des Verriegelungselementes des Kopfes führt, wird der Haltestift im Kopf verriegelt und so der Kopf am Schaft befestigt. Vorzugsweise ist bereits eine Vierteldrehung ausreichend, um eine offene Rotationsstellung des Verriegelungselements in eine geschlossene Rotationsstellung zu überführen.

Um die Höhe (in einer axialen Richtung) des Gelenkspalts anzupassen, kann optional die Unterlegscheibe zwischen Schaftoberteil und Resektionsfläche des Radius am Schaftunterteil befestigt werden.

Die Unterlegscheibe ist vorzugsweise derart ausgestaltet, dass sie nach dem Einschrauben des Gewindeankers mittels des Schafts seitlich auf das Schaftunterteil aufgeschoben werden kann. Dazu weist die Unterlegscheibe vorzugsweise eine zentrale Öffnung auf sowie eine Aussparung, die sich von dem Loch nach einer Seite bis zum Rand der Unterlegscheibe fortsetzt. Die Aussparung hat dabei vorzugsweise einen kleineren Durchmesser als das Schaftunterteil am Übergang zum Schaftoberteil, so dass die Unterlegscheibe leicht elastisch verformt, insbesondere gespannt, werden muss, um sie auf das Schaftunterteil aufschieben zu können. Um eine derartige Verformung zu erleichtern, kann die Unterlegscheibe Öffnungen aufweisen. Sobald die Unterlegscheibe ihren Bestimmungsort auf dem Schaftunterteil erreicht hat und die zentrale Öffnung der Unterlegscheibe das Schaftunterteil umschließt, entspannt sich die Unterlegscheibe, so dass die Unterlegscheibe nicht von dem Schaftunterteil herunterrutschen kann.

Die Seite der Unterlegescheibe, die zum Knochenlager hin gewendet ist, kann in einigen Fällen durch ihre Oberflächenstruktur (z.B. durch ihre Mikro- und/oder Makrostruktur, eine Beschichtung etc.) osseointegrativ ausgestaltet und auf der dem Schaft zugewandten Seite beispielsweise glatt sein.

In einigen Ausführungsformen weist das Radiusköpfchenimplantat Flächen für eine sekundäre Verankerung auf. Dazu ist das Radiusköpfchenimplantat zumindest teilweise mit einer Fläche zur sekundären Verankerung durch Einwachsen versehen. In einigen Ausführungsformen kann das Radiusköpfchenimplantat spezifische Stellen zur Osseointegration an mindestens einem der folgenden Orte aufweisen: Unterseite des Schaftoberteils, Gewindeanker, Spitze des Schaftunterteils, Unterlegscheibe.

In einigen Ausführungsformen enthält das Radiusköpfchenimplantat keine Fläche für eine sekundäre Verankerung. Damit kann der Gewindeanker primär verankert werden durch Einschrauben in den Radius, wobei der Schaft entlang seiner Längsachse beweglich bleiben kann.

In einigen Ausführungsformen besteht eine Fläche zur sekundären Verankerung am Gewindeanker. In dieser Ausführungsform kann der Gewindeanker einwachsen, wobei der Schaft jedoch entlang seiner Längsachse relativ zum Gewindeanker beweglich oder verschiebbar bleiben kann.

In einigen Ausführungsformen umfasst das Radiusköpfchenimplantat eine Fläche zur sekundären Verankerung am Schaft oder zumindest an Teilen des Schafts. Hier kann eine sekundäre Verankerung über den Schaft stattfinden. Nach Einwachsen ist dann der Schaft nicht mehr entlang seiner Längsachse beweglich.

In einigen Ausführungsformen weist das Radiusköpfchenimplantat Flächen zur sekundären Verankerung am Schaft und am Gewindeanker auf. Nach einem Einwachsen ist der Schaft nicht mehr entlang seiner Längsachse beweglich.

In einigen Ausführungsformen sind mindestens zwei, vorzugsweise alle, Teile des Radiusköpfchenimplantats aus demselben Material hergestellt.

In einigen Ausführungsformen bestehen ein, mehrere oder alle Teile des Radiusköpfchenimplantats aus einer Kobalt-Chrom-Legierung (CoCr).

In einigen Ausführungsformen bestehen ein, mehrere oder alle Teile des Radiusköpfchenimplantats aus Titan, insbesondere Titan Grad 5.

In einigen Ausführungsformen besteht der Kopf des Radiusköpfchenimplantats ganz oder teilweise aus Pyrocarbon und/oder Keramik.

In einigen Ausführungsformen besteht der Kopf aus CoCr, der Gewindeanker aus Titan (insbesondere Titan Grad 5) und der Schaft aus CoCr oder Titan (insbesondere Titan Grad 5).

In einigen Ausführungsformen besteht der Kopf teilweise oder ganz aus Pyrocarbon, wobei Schaft und Gewindeanker aus CoCr bestehen.

In einigen Ausführungsformen besteht der Kopf teilweise oder ganz aus Keramik, wobei der Schaft und/oder der Gewindeanker vorzugsweise aus Titan (insbesondere Titan Grad 5) bestehen.

Zur Verwendung mit einem Radiusköpfchenimplantat für das Capitulum humeri wird in einigen Ausführungsformen ein Kopf verwendet, der einen Kunststoff (insbesondere Polyethylen, insbesondere hochvernetztes Polyethylen, insbesondere mit Vitamin E) umfasst oder aus diesem besteht.

Beim Verwenden derselben Materialien für mehrere oder alle Teile des Implantats kann die Gefahr einer galvanischen Korrosion und eines Kaltverschweißens verringert oder vermieden werden.

In einigen Ausführungsformen weisen das erfindungsgemäße Radiusköpfchenimplantat, der Kopf, der Gewindeanker, der Schaft, die Unterlegscheibe, die Sägeschablone, das Kopf-Applikationswerkzeug, das Schaft-Implantationswerkzeug, die Ratsche, das Knochenbearbeitungswerkzeug, das Kit und/oder das Verfahren einen oder mehrere der im Voraus genannten und/oder folgenden Vorteile auf.

In einigen Ausführungsformen können durch die Erfindung die Artikulationsflächen des Radiusköpfchens mit dem Capitulum humeri und/oder des proximalen Radioulnargelenks wiederhergestellt werden. Dabei können Schmerzen gemindert oder dauerhaft eliminiert und/oder Beweglichkeit (wieder) ermöglicht werden. Durch die Erfindung können in einigen Fällen eine Primär- und Sekundärstabilität, lange Standzeit und/oder geringer oder kein Abrieb erreicht werden und/oder ungewünschte Reaktionen zwischen Gewebe und Implantat vermieden werden. Die Erfindung kann häufig die richtige Gelenkspannung erreichen und zur Minimierung oder Verhinderung von Luxationen oder Subluxationen etc. beitragen. Vorzugsweise ist die Erfindung biokompatibel und wird der Biomechanik gerecht.

In einigen Fällen kann durch die Passgenauigkeit der Sägeschablone, insbesondere durch die Größe der Schablone bezogen auf den Gelenkspalt, das Capitulum, den Radiuskopf und/oder die mediale Tiefe der Trochlea, und durch die achsgenaue Ausrichtung bezogen auf den Radiusschaft, die Platte als Führung der Schnittebene in der xy-Ebene dienen. Durch die Öffnungen (L/R) kann diese Schnittebene temporär für die Dauer der Resektion am distalen Ende des Humerus (Capitulum humeri) durch einen Kirschnerdraht oder Ähnliches fixiert werden. Die Schnittebene an dem resezierten Radiusschaft weist somit in Bezug auf das Capitulum humeri und die restliche Anatomie die optimale dreidimensionale Ausrichtung auf und hat durch die Auswahl der richtigen Größe auch durch die Größenabstimmung zu dem jeweiligen Implantat (Radiusköpfchen) den entsprechenden Abstand zwischen Capitulum humeri und reseziertem Radiusschaft. Dies bedeutet, dass dadurch das im folgenden Operationsverlauf ausgewählte und eingesetzte Radiusköpfchenimplantat anatomisch ausgerichtet ist und den richtigen Abstand zum Capitulum aufweist (kein over-/understuffing) und die Artikulation zum Radioulnargelenk hergestellt wird.

In einigen Ausführungsformen sind ein oder mehrere der folgenden anatomisch beidseitig verwendbar: Kopf, Gewindeanker, Schaft, Unterlegescheibe, Radiusköpfchenimplantat, Sägeschablone, Kopf-Applikationswerkzeug, Schaft-Implantationswerkzeug, Ratsche, Knochenbearbeitungswerkzeug.

Im Folgenden wird das hierin offenbarte Verfahren anhand bevorzugter Ausführungsformen des erfindungsgemäßen Radiusköpfchenimplantats unter Bezugnahme auf die angehängte Zeichnung beschrieben. Die Erfindung ist jedoch nicht auf diese Ausführungsformen beschränkt. In den Figuren gilt:
- Fig. 1: zeigt das erfindungsgemäße Radiusköpfchenimplantat;
- Fig. 2: zeigt den Kopf für ein erfindungsgemäßes Radiusköpfchenimplantat;
- Fig. 3: zeigt den hierin offenbarten Kopf in drei weiteren Ansichten;
- Fig. 4: zeigt den hierin offenbarten Schaft;
- Fig. 5: zeigt den hierin offenbarten Gewindeanker;
- Fig. 6: zeigt den Gewindeanker, in den ein Schaft eingesetzt ist;
- Fig. 7: zeigt die hierin offenbarte Sägeschablone und eine Größenlehre;
- Fig. 8: zeigt die Sägeschablone eingesetzt in einen Gelenkspalt;
- Fig. 9: zeigt das hierin offenbarte Knochenbearbeitungswerkzeug;
- Fig. 10: zeigt das hierin offenbarte Schaft-Implantationswerkzeug sowie den Schaft mit aufgesetztem Gewindeanker;
- Fig. 11: zeigt die hierin offenbarte Ratsche und den in den Knochen eingesetzten Schaft mit Gewindeanker;
- Fig. 12: zeigt das hierin offenbarte Kopf-Applikationswerkzeug;
- Fig. 13: zeigt das Radiusköpfchenimplantat mit seinem Verriegelungselement in zwei unterschiedlichen Rotationsstellungen;
- Fig. 14: zeigt das Verriegelungselement des Kopfes;
- Fig. 15: zeigt das erfindungsgemäße Radiusköpfchenimplantat, das in einen Radius eingesetzt ist und die hierin offenbarte Unterlegscheibe; und
- Fig. 16: zeigt das Radiusköpfchenimplantat mit der Unterlegscheibe in einer weiteren Darstellung.

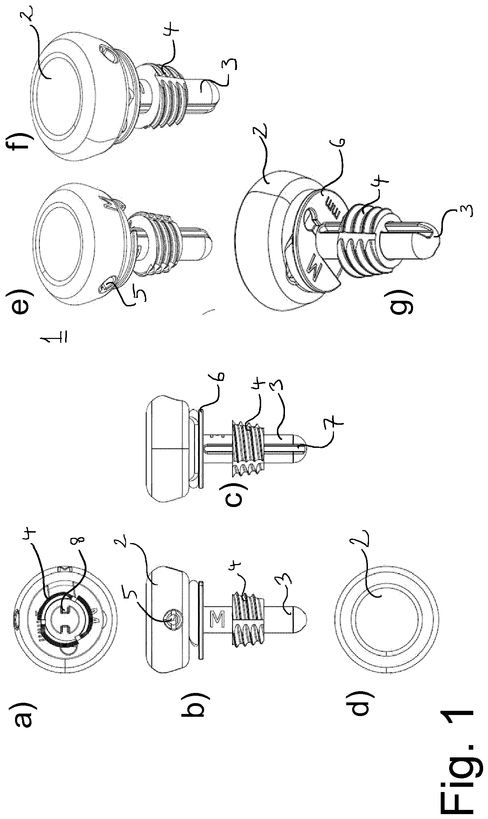

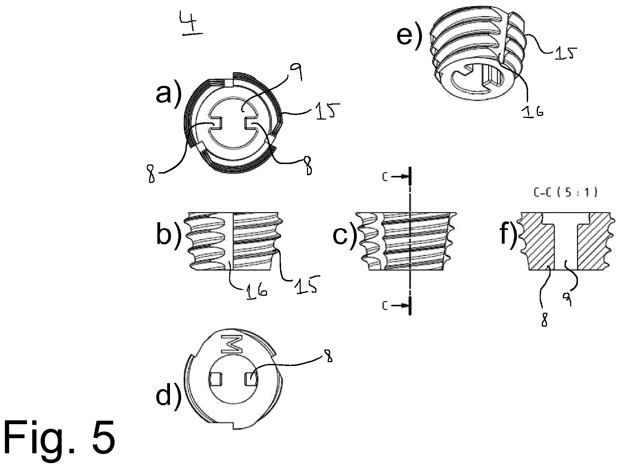

**Fig. 1** zeigt ein Radiusköpfchenimplantat 1 mit einem Kopf 2, einem Schaft 3 und einem Gewindeanker 4. In Fig. 1a und 1d sind Ansichten von unten beziehungsweise oben zu sehen. In Fig. 1b und 1c sind seitliche Ansichten gezeigt und Fig. 1e, 1f und 1g zeigen perspektivische Darstellungen. In der seitlichen Ansicht in Fig. 1b ist auch ein Verriegelungselement 5 zu erkennen sowie die optionale Bezeichnung "M", die in diesem Fall auf die Größe des Implantats hinweist. In Fig. 1g ist weiter eine optionale Unterlegscheibe 6 zu sehen, die auf den Schaft 3 aufgeschoben ist. In Fig. 1c, 1e, 1f ist eine von zwei (könnten auch mehr als zwei sein) optionalen Rinnen 7 im Schaft 3 zu sehen, die z. B. mit zwei Nasen 8 in einer Durchgangsöffnung 9 (siehe Fig. 5f) des Gewindeankers 4 zusammenwirkt und eine Linearführung ergibt, die eine axiale Verschiebung des Schafts 3 im Gewindeanker 4 erlaubt. Die Rinnen 7 und die Nasen 8 ermöglichen zudem eine Übertragung eines Drehmoments vom Schaft 3 auf den Gewindeanker 4, was beim Eindrehen des Radiusköpfchenimplantats 1 oder dessen Gewindeankers 4 in einen Radiusknochen von Nutzen sein kann.

Rinne(n) 7 und Nase(n) 8 können alternativ vertauscht sein, so dass die Rinne(n) 7 nicht auf dem Schaft 3 sondern auf dem Gewindeanker 4 vorgesehen ist/sind, und umgekehrt.

Anstelle der Rinne(n) 7 und der Nase(n) 8 können andere Element zum Herstellen eines Form- und/oder Kraftschlusses zwischen Schaft 3 und Gewindeanker 4 vorgesehen sein.

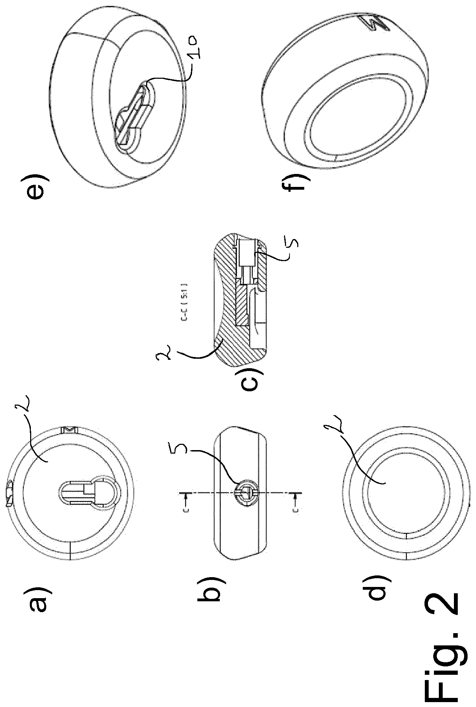

**Fig. 2** zeigt den Kopf 2 des Radiusköpfchenimplantats 1 in einer Ansicht von unten (Fig. 2a), von der Seite (Fig. 2b) und oben (Fig. 2d).

In Fig. 2e und 2f ist eine perspektivische Ansicht des Kopfes 2 von schräg unten bzw. schräg oben dargestellt. Fig. 2c zeigt einen Schnitt durch eine Längsachse des Verriegelungselements 5.

Insbesondere Fig. 2a und 2e zeigen deutlich eine Öffnung 10 des Kopfes 2.

Ein erster Anteil der Öffnung 10 weist einen größeren Durchmesser auf, und ein zweiter Anteil weist einen geringeren Durchmesser oder eine geringe Breite (z. B. geringer als der Durchmesser des ersten Anteils) auf. Der Anteil der Öffnung 10 mit dem größeren Durchmesser hat hier eine kreisförmige Begrenzung. Ein sich anschließendes Rechteck (das hier exemplarisch wieder in einen kleineren Kreis oder Halbkreis mündet) weist einen geringeren Durchmesser oder eine geringere Breite auf.

Erster Anteil und zweiter Anteil können gemeinsam optional die Form eines Schlüssellochs haben.

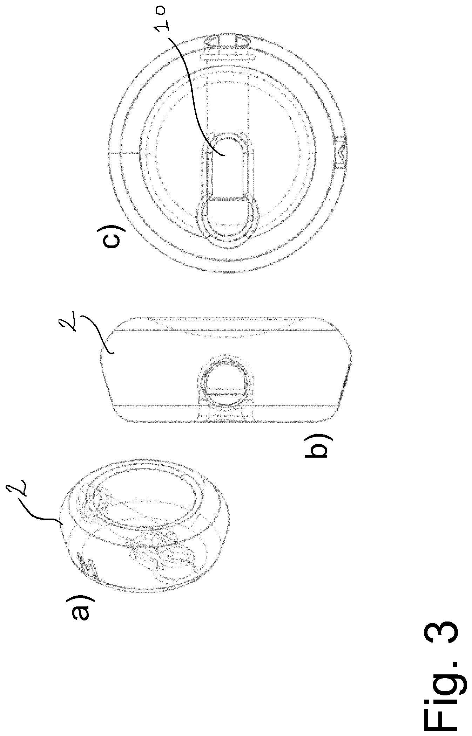

In **Fig. 3a bis 3c** ist erneut der Kopf 2 gezeigt mit der Öffnung 10 und der Öffnung zum Einführen des Verriegelungselements 5.

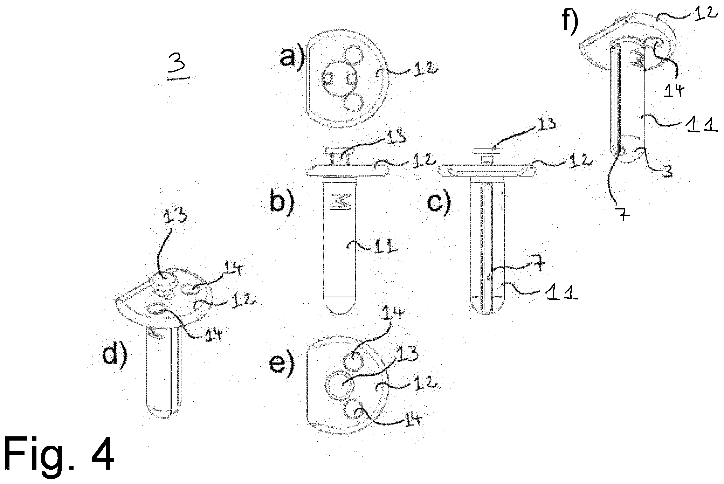

**Fig. 4** zeigt mehrere Darstellungen des Schafts 3 mit einem Schaftunterteil 11 und einem Schaftoberteil 12 sowie der optionalen Rinne 7. Ein Haltestift 13 des Schaftoberteils 12 zeigt hier einen bezogen auf den unteren Anteil breiteren, oberen Anteil mit einer optional kreisförmigen Fläche oder Grundfläche. Der untere Anteil des Haltestifts 13 liegt zwischen dem oberen Anteil und der proximalen Oberfläche des Schaftoberteils 12. Der untere Anteil des Haltestifts 13 weist hier rein optional einen eckigen und keinen runden Querschnitt auf.

Zwei Öffnungen 14 sind in das Schaftoberteil 12 eingebracht. Diese können verwendet werden, um ein Drehmoment von einem Werkzeug auf den Schaft 3 zu übertragen, beispielsweise um den Schaft 3 mit seinem Gewindeanker 4 in den Radius einzudrehen.

Der Schnitt durch das Schaftoberteil 12 in einer Ebene senkrecht zur Längsachse des Schaftunterteils 11 ergibt im Beispiel der Fig. 4 eine Form, die durch einen (Teil-)Kreisbogen (alternativ einen nichtrunden, etwa ovalen (Teil-)Bogen), dessen Enden durch eine gerade Linie verbunden sind, definiert ist. Mit anderen Worten hat der Schnitt die Form eines Kreisabschnitts. Durch diese so abgeflachte Form des Schaftoberteils 12 ist das Einsetzen des Schafts 3 in den Radius insbesondere bei minimalinvasiven Operationen erleichtert. Der Schaft 3 kann so vorteilhaft auch bei einem engen operativen Zugang in den Radius eingeführt werden.

Statt zwei Öffnungen 14 können mehrere Öffnungen 14 vorgesehen sein. Alternativ würden eine Öffnung 14 und ein Anschlag oder dergleichen genügen, um den gewünschten Form- und/oder Kraftschluss für das Werkzeug zu gewährleisten.

In **Fig. 5** ist der Gewindeanker 4 von oben (Fig. 5d), unten (Fig. 5a) und der Seite (Fig. 5b und 5c) gezeigt. Fig. 5f zeigt eine Schnittdarstellung, und in Fig. 5e ist der Gewindeanker 4 perspektivisch von schräg unten gezeigt. Die äußere Form des Gewindeankers 4 entspricht im Wesentlichen einem Kegelstumpf, auf den ein Gewinde 15 aufgebracht ist.

Das Gewinde 15 des Gewindeankers 4 ist selbstschneidend, insbesondere auch bei Linksdrehung, um ein Entfernen des Radiusköpfchenimplantats zu erleichtern.

Das Gewinde 15 weist optional an verschiedenen (hier rein exemplarisch drei) Positionen des Umfangs Unterbrechungen 16 auf, um das beim Gewindeschneiden freigesetzte Knochenmaterial abzuführen und/oder den Span zu brechen.

In Fig. 5 ist zudem die Durchgangsöffnung 9 des Gewindeankers 4 dargestellt. Auch die in der Durchgangsöffnung 9 optional vorhandenen Nasen 8 zum Eingreifen in die Rinnen 7 des Schaftunterteils 11 sind hier zu sehen. Die Nasen 8 lassen eine axiale Verschiebung des Schafts 3 relativ zum Gewindeanker 4 zu. Darüber hinaus kann über die Rinnen 7 und die Nasen 8 ein Drehmoment vom Schaft 3 auf den Gewindeanker 4 übertragen werden.

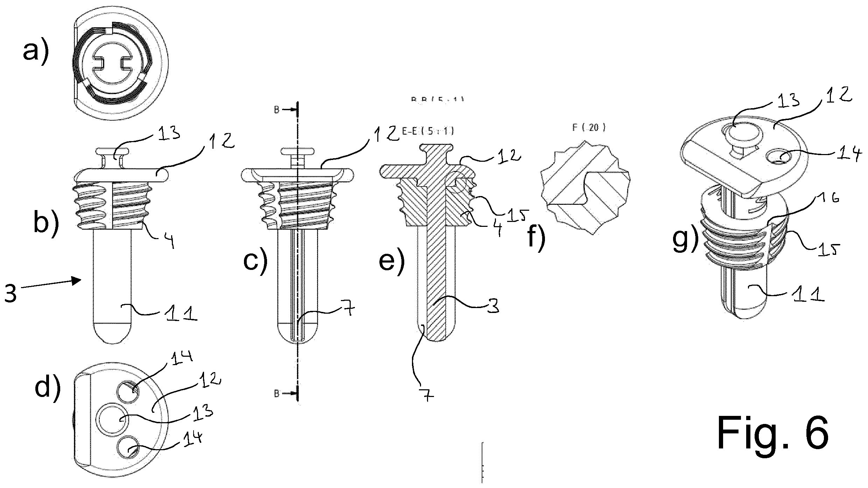

**Fig. 6** zeigt Darstellungen des Schafts 3 mit aufgesetztem Gewindeanker 4 von oben (Fig. 6d), unten (Fig. 6a), von der Seite (Fig. 6b und 6c), im Schnitt durch die Längsachse (Fig. 6e), mit einer Detaildarstellung (Fig. 6f) und in einer perspektivischen Darstellung von schräg oben (Fig. 6g) .

Der Gewindeanker 4 ist in Fig. 6b bis 6f optional lösbar und verliersicher auf dem Schaft 3 verklemmt oder verrastet. Dazu greifen die in Fig. 6e gezeigten und in Fig. 6f vergrößert dargestellten Anteile des Schaftunterteils 11 am Übergang zum Schaftoberteil 12 in den proximalen Teil des Gewindeankers 4 ein. Die verliersichere, lösbare Befestigung des Gewindeankers 4 am Schaft 3 verhindert beim Einsetzen des Schafts 3 ein ungewolltes Herausfallen des Gewindeankers 4, was eine Operation potentiell verlängern könnte oder gar dazu führen könnte, dass der Gewindeanker 4 unsteril wird. Dies wird durch die gezeigte lösbare Verliersicherung verhindert.

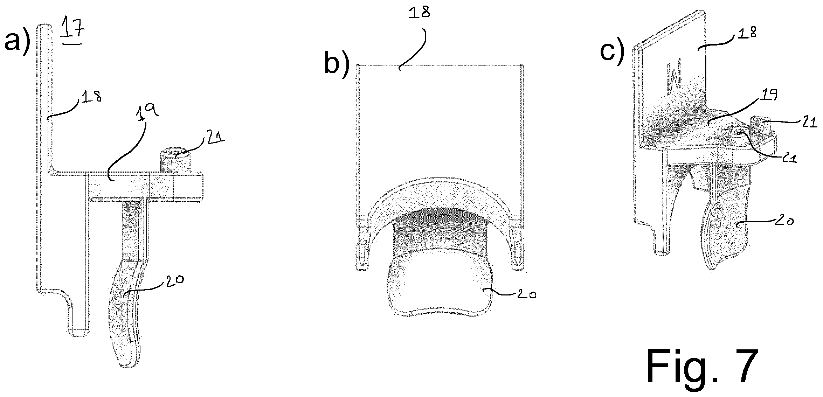

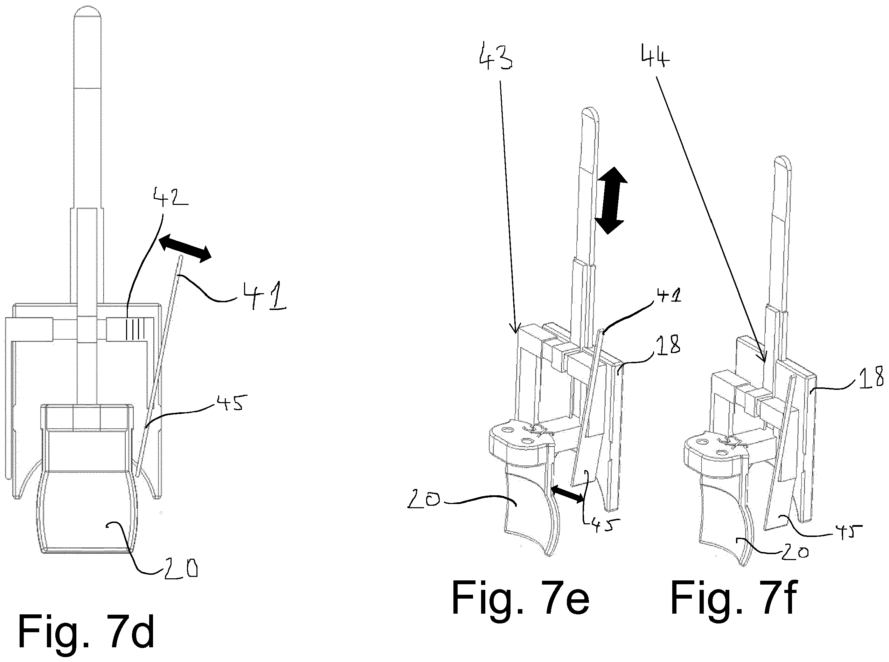

In **Fig. 7** ist die hierin offenbarte Sägeschablone 17 von der Seite (Fig. 7a), von distal (Fig. 7b) und perspektivisch von schräg oben (Fig. 7c) gezeigt.

Die Sägeschablone 17 weist eine Platte 18 und eine über ein Verbindungselement 19 parallel zur Sägeschablone 17 angebrachte Zunge 20 auf. Zudem sind in Fig. 7a und Fig. 7c Durchgangsöffnungen 21 gezeigt, die sich nach außen hin in einem kurzen Hohlzylinder fortsetzen. Die Durchgangsöffnungen 21 dienen dem Einführen von Drähten, mit denen die Sägeschablone 17 am Humerus fixiert werden kann. Dabei wird nur die eine Durchgangsöffnung 21 verwendet, wenn am linken Arm operiert wird, und nur die andere Durchgangsöffnung 21, wenn am rechten Arm operiert wird.

Um Verwechslungen zwischen ihnen zu vermeiden, sind die Durchgangsöffnungen 21 auf dem Verbindungselement 19 optional bezeichnet, etwa mit R und L.

Die Platte 18 hat zum Radiusköpfchen hin eine, vorzugsweise kreisbogenförmige, Begrenzung, so dass sich die Platte 18 möglichst großflächig am Radiusköpfchen abstützen kann. Die Zunge 20 weist optional in Richtung der Platte 18 eine Konvexität auf, die dazu dient, besser auf der konkaven Gelenkfläche des Radiusköpfchens aufzuliegen.

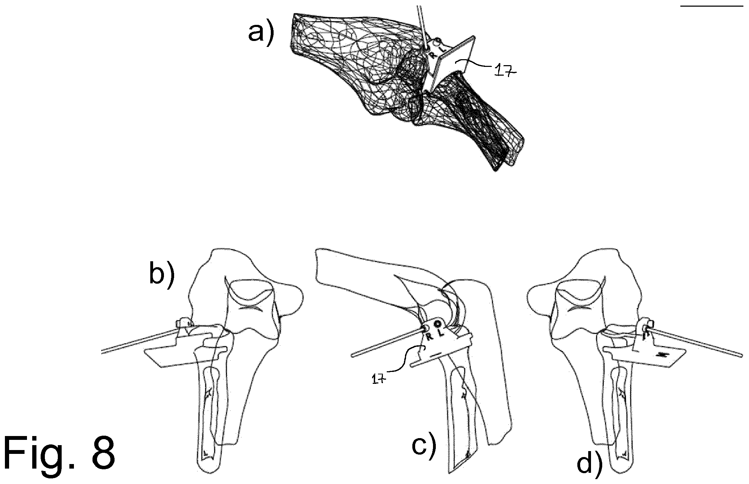

Die Sägeschablone 17 wird vor der Implantation samt ihrer Zunge 20 in den Gelenkspalt eingeführt, wobei die kreisbogenförmige untere Begrenzung der Sägeschablone 17 seitlich auf dem Radiusköpfchen aufliegt. Daraufhin wird bewertet, ob die Sägeschablone 17 auf das Radiusköpfchen ausreichend genau passt oder ob eine andere Sägeschablone 17 in einer anderen Größe verwendet werden sollte. Dies kann haptisch oder visuell beurteilt werden. In diesem Zusammenhang wird die Sägeschablone 17 zur Größenbestimmung des Radiusköpfchens verwendet. Wenn die richtige Größe gefunden ist, so kann die entsprechende Sägeschablone 17 in den Gelenkspalt ein- und auf den Radius aufgesetzt und mit einem Draht befestigt werden, wie in **Fig. 8a bis 8d** gezeigt ist. Daraufhin kann die distale Oberfläche der Platte 18 zur Führung etwa einer oszillierenden Säge verwendet werden, um das Radiusköpfchen zu resezieren.

In einigen Ausführungsformen (Fig. 7d bis 7f) kann die Sägeschablone 17 im Zusammenhang mit einer Größenlehre 43 verwendet werden. Die Größenlehre 43 kann dabei beispielsweise mit Hilfe eines Schlittens 44 der Größenlehre 43, der in der Platte 18 der Sägeschablone 17 geführt ist, auf das Radiusköpfchen aufgeschoben werden. Dabei wird ein Arm 45 der Größenlehre 43 durch Berührung mit dem seitlichen Rand des Radiusköpfchens ausgelenkt, so dass ein mit dem Arm 45 verbundener Zeiger 41 ausgelenkt wird. Die Auslenkung des Zeigers 41 kann an einer auf der Größenlehre 43 aufgebrachten Skala 42 abgelesen werden. Dabei entspricht ein Wert der Skala 42 einer bestimmten Größe eines Radiusköpfchens, so dass entsprechende Implantatgrößen und hierzu größenmäßig passende Werkzeuge anhand des Skalenwertes ausgewählt werden können.

Die Größe des resezierten Radiusköpfchens kann mit einer nicht gezeigten Schablone, die kreisförmige Vertiefungen in verschiedenen Durchmessern aufweist, überprüft werden.

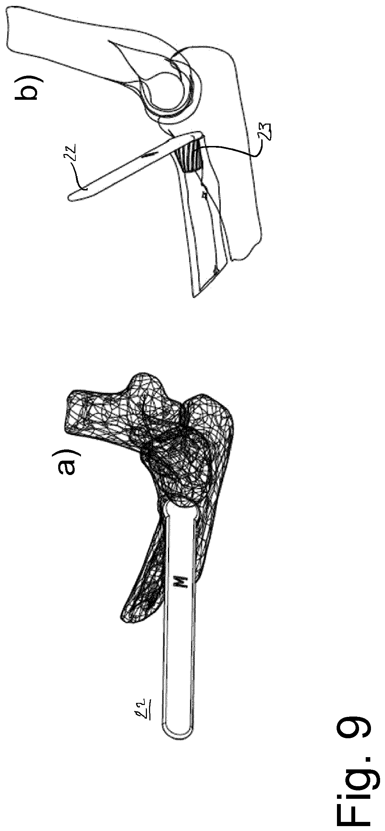

Nach der Resektion kann ein Knochenbearbeitungswerkzeug 22 mit einem Raspelelement 23 in den Markraum des Radius eingebracht werden (siehe **Fig. 9a und 9b**)**.** Durch Schwenken eines Stiels 24 des Knochenbearbeitungswerkzeugs 22 wird der Markraum durch Raspeln erweitert. Dazu weist die Oberfläche des Raspelelements 23 z. B. Einkerbungen oder Zähne auf.

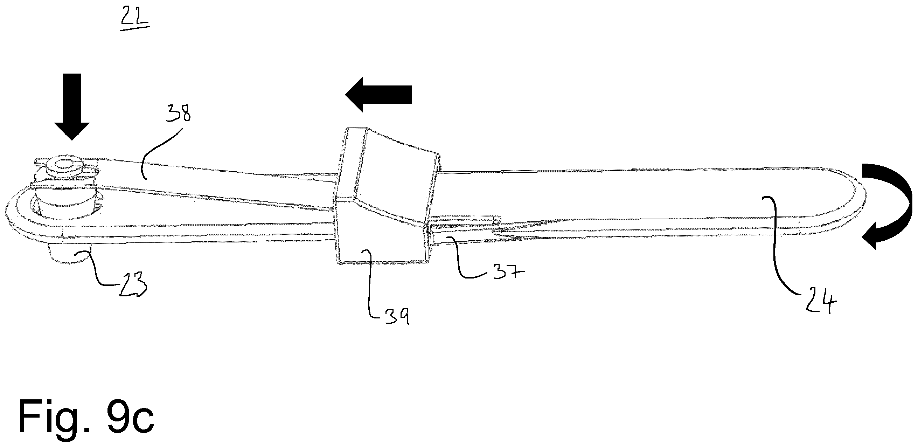

In einer Ausführungsform (siehe **Fig. 9c**) des Knochenbearbeitungswerkzeugs 22 mit Stiel 24 weist dieses einen Schieber 39 auf, der entlang einer Blattfeder 38 verschoben werden kann. An der Blattfeder 38 ist das Raspelelement 23 befestigt. Durch Verschieben des Schiebers 39 kann das Raspelelement 23 durch den Stiel 24 hindurchgedrückt werden. Der Schieber 39 ist optional mit einer Verliersicherung 37 ausgestattet, hier optional als Rastzunge ausgeführt, die verhindert, dass der Schieber 39 vom Raspelelement 23 weg vom Stiel 24 rutscht.

Der Stiel 24 des Knochenbearbeitungswerkzeugs 22 kann als Ratsche ausgeführt sein.

Das Raspelelement 23 kann austauschbar oder auswechselbar gestaltet sein, so dass z. B. Raspelelemente 23 in verschiedenen Größen mit dem Knochenbearbeitungswerkzeug 22 verwendet werden können.

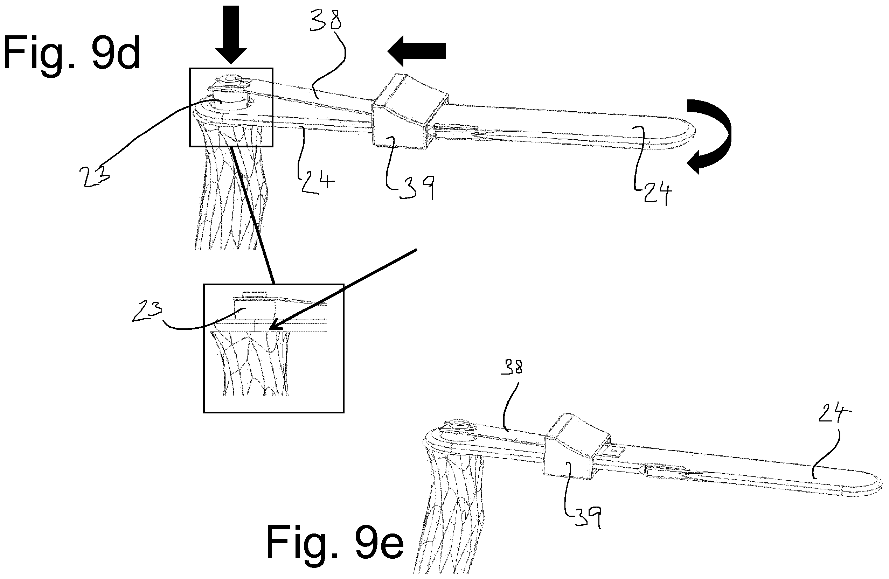

In **Fig. 9d und 9e** wird die Verwendung des Knochenbearbeitungswerkzeugs 22 gezeigt. Das Knochenbearbeitungswerkzeug 22 liegt dabei mit seinem Stiel 24 auf der Resektionsebene des Radius eben auf. Die Drehachse des Raspelelements 23 steht dabei senkrecht auf der Resektionsebene. Durch Drehbewegungen des Knochenbearbeitungswerkzeugs 22 schneidet sich das konische, durch die Blattfeder 38 gefederte Raspelelement 23 in den Markraum des Radius ein.

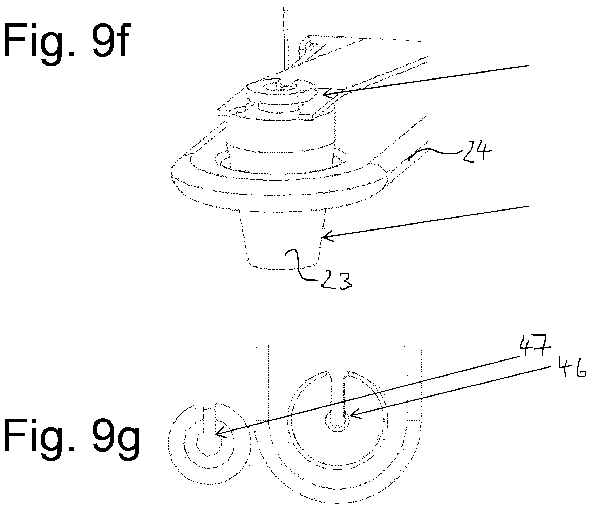

**Fig. 9f und 9g** zeigen Detailansichten des Knochenbearbeitungswerkzeugs 22. Dabei ist zu sehen, dass in die Blattfeder 38 optional eine Verliersicherung 46 integriert ist, die in das Raspelelement 23 eingreift und auch zum Antrieb des Raspelelements 23 dient. Die Verliersicherung 46 der Blattfeder 38 ist in Fig. 9g optional als eine Zunge ausgeführt, die in einem schmalen Steg von der übrigen Blattfeder 38 weg verläuft und sich an ihrem freien Ende zu einer Kreisform verbreitert. Die Zunge wird zur Verwendung des Knochenbearbeitungswerkzeugs 22 in das Raspelelement 23 eingeführt und überträgt Drehmomente des Stiels 24 auf das Raspelelement 23. Das Raspelelement 23 weist dazu optional eine der Verliersicherung 46, insbesondere der Zunge, entsprechende Vertiefung 47 auf.

Das Raspelelement 23 ist vorzugsweise konisch ausgeführt und schneidet sich durch Drehbewegungen des Stiels 24 und den Anpressdruck von Blattfeder 38 und Schieber 39 in eine gewünschte, größenabhängige Tiefe in den Radius ein.

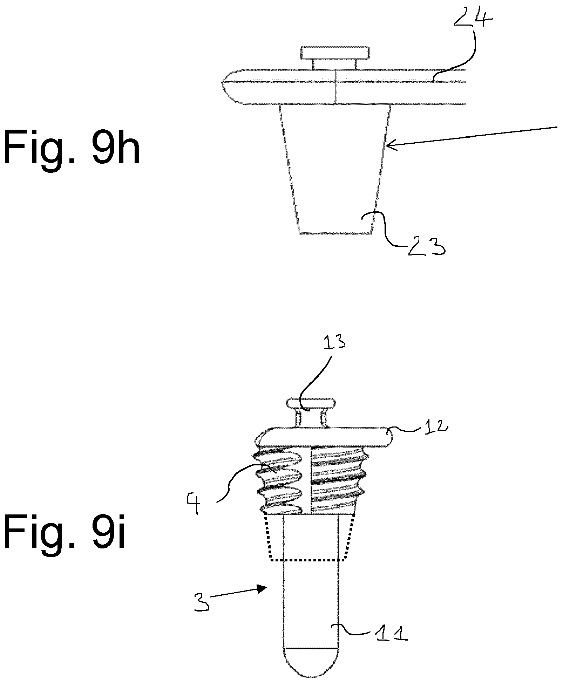

Das Raspelelement 23 ist dabei vorzugsweise auf die Abmessungen des Gewindeankers 4 abgestimmt, wie in **Fig. 9h und 9i** zu sehen ist.

In einigen Ausführungsformen kann das Raspelelement 23 zwei oder mehr Stufen aufweisen. Das konische Raspelelement 23 schafft beim Verwenden des Knochenbearbeitungswerkzeugs 22 durch Entfernen von Knochenmaterial Platz im Radius für den Gewindeanker 4 und den Schaft 3. Das Raspelelement 23 schneidet zudem den Platz im Radius frei bis zum Gewindegrund des Gewindeankers 4, so dass ein Einschrauben des Gewindeankers 4 ermöglicht wird (siehe die gestrichelte Linie in Fig. 9i).

Nach der Bearbeitung des Knochens mit dem Knochenbearbeitungswerkzeug 22 ist der Knochen bereit zum Einsetzen des Schafts 3 mit dem optional verliersicher aufgesetzten Gewindeanker 4.

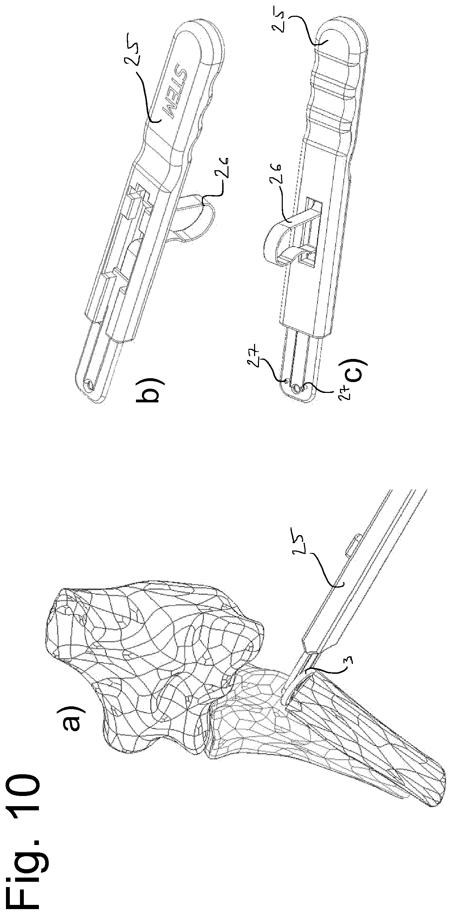

Das Einsetzen erfolgt mit einem Schaft-Implantationswerkzeug 25, das in **Fig. 10a bis 10c** dargestellt ist. Das Schaft-Implantationswerkzeug 25 weist ein optional elastisches Element 26 auf, das zur Fixierung des Haltestifts 13 am Knochenbearbeitungswerkzeug 22 dient. Das Knochenbearbeitungswerkzeug 22 weist zudem zwei oder mehr Stifte 27 auf, die in die Öffnungen 14 des Schaftoberteils 12 eingesetzt werden können, um den Schaft 3 sicher und präzise in den Radius einführen zu können. Die Situation nach dem Einführen des Schafts 3, wobei der Schaft 3 noch immer am Knochenbearbeitungswerkzeug 22 befestigt ist, ist in Fig. 10a gezeigt.

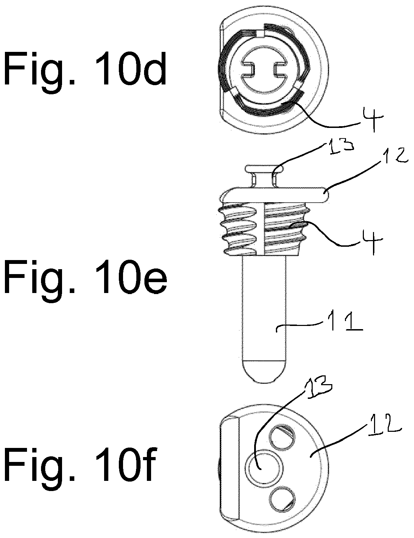

In **Fig. 10d bis 10f** ist zu erkennen, dass das Schaftoberteil 12 in der Ansicht von oben (Fig. 10f) und von unten (Fig. 10d), z. B. im Sinne eines Kreisabschnitts, seitlich optional abgeflacht ist. Zudem ist in Fig. 10e zu erkennen, dass das Schaftoberteil 12 an seiner flachen Seite nach proximal abgeschrägt sein kann, was ein minimalinvasives Einkippen des Schafts 3 in den Radius zusätzlich erleichtern kann.

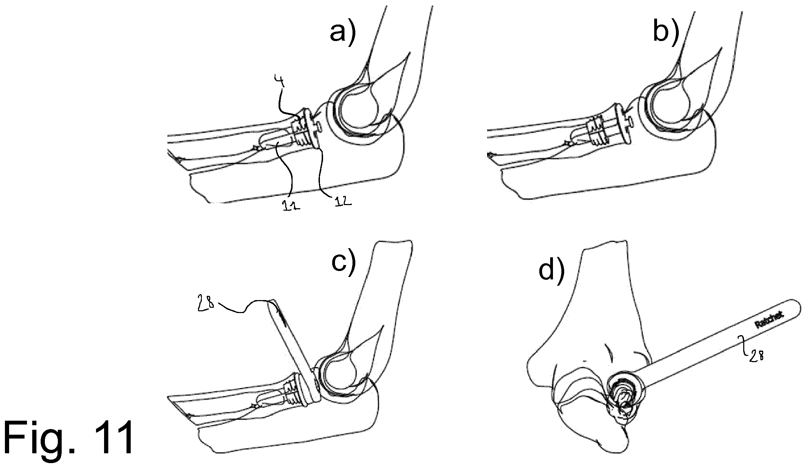

Der eingesetzte Schaft 3 mit Gewindeanker 4 wird in **Fig. 11a** gezeigt, der Gewindeanker 4 liegt hier noch am Schaftoberteil 12 an und ist noch nicht in den Knochen geschraubt. An das Schaftoberteil 12 kann daraufhin eine Ratsche 28 angelegt werden, was in **Fig. 11c und 11d** gezeigt wird. Durch Drehen der Ratsche 28 kann ein Drehmoment über den bedingt durch Ratsche 28 rotierenden Schaft 3 auf den Gewindeanker 4 übertragen werden, so dass der Gewindeanker 4 tiefer in den Radius eingeschraubt werden kann. Da der Schaft 3 jedoch axial frei beweglich im Gewindeanker 4 vorliegt (durch Eingriff oder Führung zwischen Rinnen 7 und Nasen 8), muss sich durch das Einschrauben des Gewindeankers 4 nicht notwendigerweise die Position des Schafts 3 verändern.

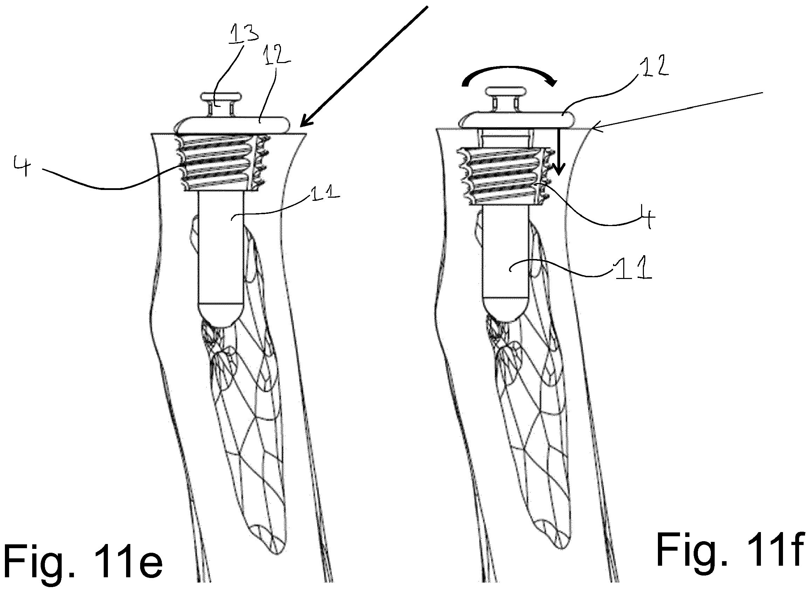

In **Fig. 11e und 11f** ist dargestellt, dass das Schaftoberteil 12 optional ausgestaltet ist, um auf der Resektionsfläche (auf sie ist in Fig. 11e und 11f mit Pfeilen hingewiesen) des Radius aufzuliegen und so eine gewünschte Drehachse des Schaftunterteils 11 mit Gewindeanker 4 oder zumindest die Neigung der Drehachse festzulegen.

Fig. 11e zeigt den Schaft 3 mit Gewindeanker 4 in einer initialen Stellung nach dem Einführen. Fig. 11f zeigt, dass sich der Gewindeanker 4 - bedingt durch die Drehung des Schafts 3 - nach distal, also in den Radius hinein, geschraubt hat.

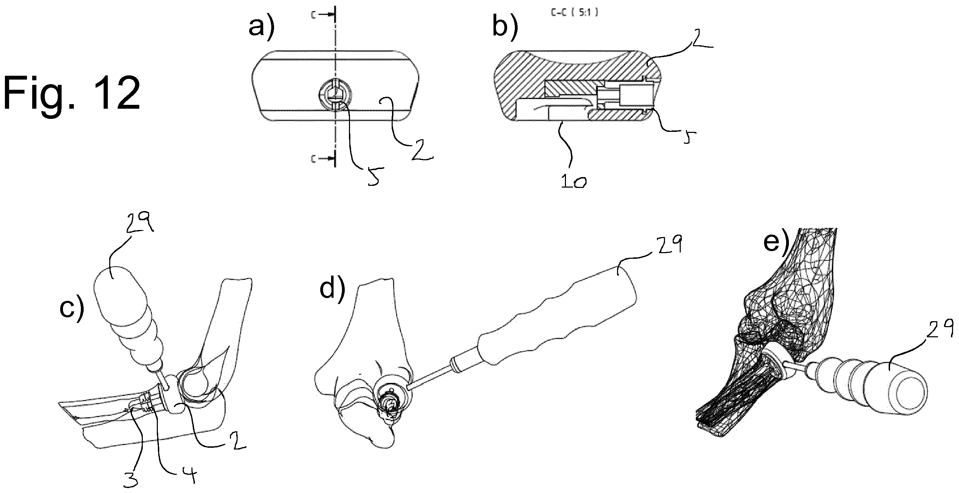

In **Fig. 12** wird ein Kopf-Applikationswerkzeug 29 gezeigt, mit dem der Kopf 2 auf den eingesetzten Schaft 3 aufgesetzt und/oder nach seinem Aufsetzen auf den Schaft 3 mit diesem verriegelt werden kann. Das Kopf-Applikationswerkzeug 29 greift dazu an dem Verriegelungselement 5 an.

In dem in Fig. 12 gezeigten Beispiel weist das Verriegelungselement 5 optional einen Innensechskant auf, in den das Kopf-Applikationswerkzeug 29 eingreift. Andere geometrische Formen als der Innensechskant sind ebenfalls von der vorliegenden Erfindung umfasst.

Das Kopf-Applikationswerkzeug 29 wird so bewegt, dass der Haltestift 13 in die Öffnung 10 des Kopfes 2 eingeführt wird bzw. der Kopf 2 mit seiner Öffnung 10 auf den Haltestift 13 aufgeschoben wird, insbesondere in einer axialen Richtung des Haltestifts 13.

Danach wird der Kopf 2, insbesondere in einer radialen Richtung, weiterbewegt, bis sich der Haltestift 13 in dem schmaleren Anteil der Öffnung 10 befindet, der Haltestift 13 axial also nicht mehr vom Kopf 2 abgezogen werden kann.

In dieser Position wird der Haltestift 13 mit dem Kopf 2 verriegelt. Dies geschieht durch ein Drehen des Verriegelungselements 5 mittels des Kopf-Applikationswerkzeug 29 von einer offenen zu einer geschlossenen Rotationsstellung.

In der geschlossenen Rotationsstellung verhindert z. B. ein Riegel 30, dass der Haltestift 13 wieder in den breiteren Teil der Öffnung 10 zurück verrutscht. Damit ist der Kopf 2 gegenüber dem Schaftoberteil 12 und somit auch gegenüber dem Schaft 3 fixiert.

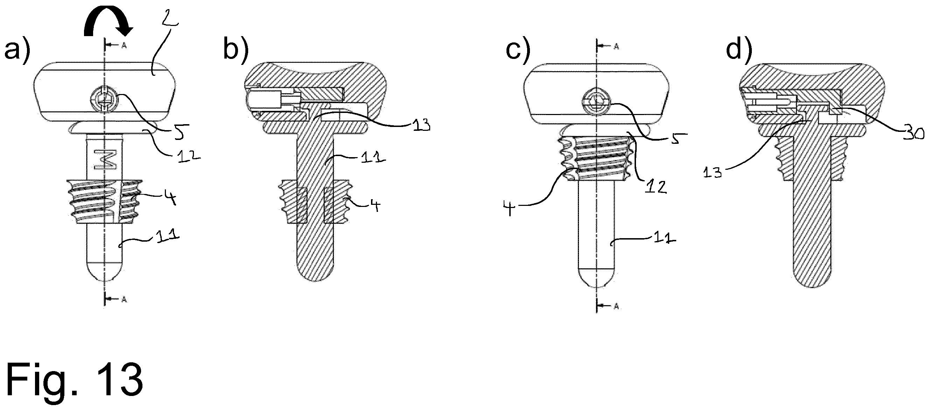

In **Fig. 13** sind die beiden Rotationsstellungen des Verriegelungselements 5 deutlich zu sehen, wobei die Stellung des Gewindeankers 4 relativ zum Schaft 3 nicht zu beachten ist.

**Fig. 13a und 13b** zeigen die offene Rotationsstellung, während Fig. 13c und 13d die geschlossene Rotationsstellung zeigt.

In **Fig. 13d** ist in einem Schnitt gezeigt, dass der Riegel 30 dem Haltestift 13 im Weg ist bzw. ihn in seiner Bewegungsfreiheit begrenzt. Somit ergibt sich ein Formschluss, der verhindert, dass der Haltestift 13 von dem schmaleren in den breiteren Teil der Öffnung 10 (hier von links nach rechts) rutscht. Aufgrund der Begrenzung kann der Haltestift 13 nicht mehr aus dem Kopf 2 entfernt werden. In der offenen Rotationsstellung ist der Riegel 30 nach oben rotiert und gibt den breiteren Anteil der Öffnung 10 frei (Fig. 13a und 13b). Dadurch ist es möglich, den Haltestift 13 durch den breiteren Anteil der Öffnung 10 in den Kopf 2 einzubringen oder aus diesem zu entfernen.

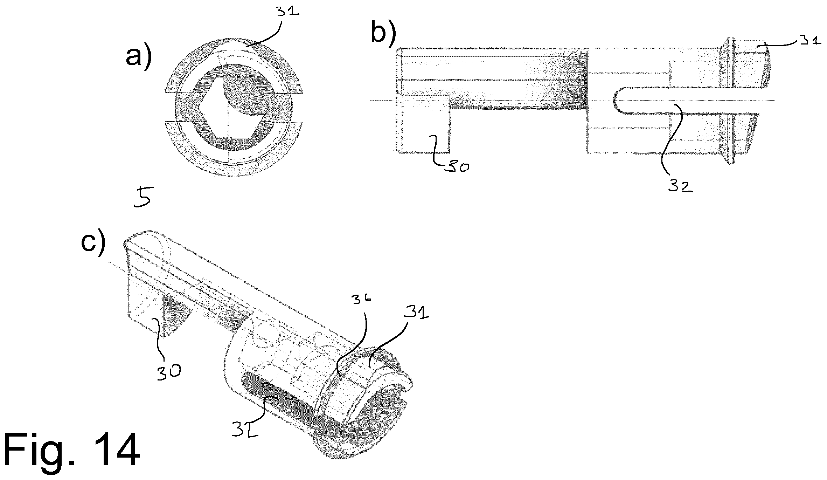

**Fig. 14** zeigt das Verriegelungselement 5. Deutlich zu sehen ist, dass das Verriegelungselement 5 einen optionalen Innensechskant aufweist, an den das Kopf-Applikationswerkzeug 29 ansetzen kann, um ein Drehmoment auf das Verriegelungselement 5 zu übertragen.

Ein optionales Rastelement 31 des Verriegelungselements 5 kann mit einer entsprechenden Vertiefung des Kopfes zusammenwirken, so dass das Verriegelungselement 5 in einer offenen und/oder einer geschlossenen Rotationsstellung einrasten kann.

Zudem weist das Verriegelungselement 5 optional eine ringförmige Verliersicherung 36 auf, die z. B. in einer Nut des Kopfes 2 verrastet.

Ein optionaler Schlitz 32 im Verriegelungselement 5 kann die für das Einrasten nötige Elastizität bereitstellen. Zudem kann der Schlitz 32 vorzugsweise genutzt werden, um das Verriegelungselement 5 mit einem Schraubendreher zu drehen.

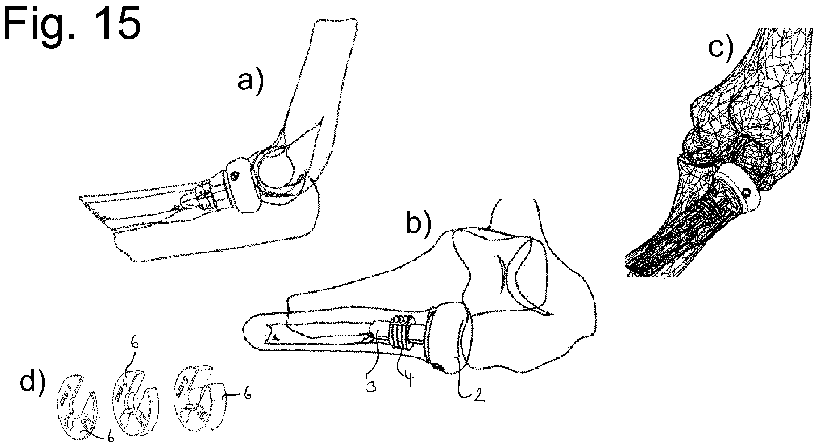

**Fig. 15** zeigt Unterlegscheiben 6 und ihre Verwendung. Falls nach Einbringen des Radiusköpfchenimplantats 1 festgestellt wird, dass der Gelenkspalt noch zu weit ist, wie dies in Fig. 15a und 15b durch einen Spalt zwischen Radius und Unterseite des Kopfs 2 angedeutet ist, so kann korrigierend eine Unterlegscheibe 6 in diesen Spalt zwischen Schaftoberteil 12 und Radius eingebracht werden. Die Unterlegscheiben 6 können in verschiedenen Stärken bereitgestellt werden, beispielsweise mit einer Dicke von 1, 3 und 5 mm.

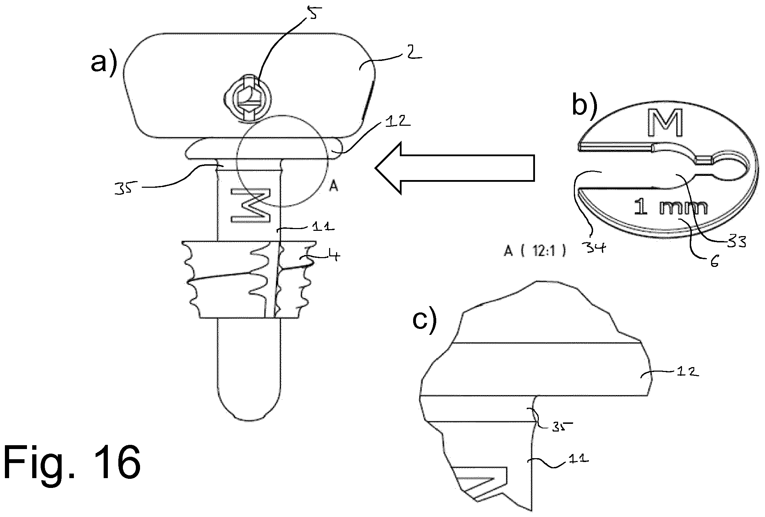

In **Fig. 16a** ist ein Radiusköpfchenimplantat 1 gezeigt, das am Schaftunterteil 11 optional eine z. B. umlaufende Nut 35 zum Aufschieben der Unterlegscheibe 6 auf das Schaftunterteil 11 aufweist. Die Nut 35 kann ein axiales Verrutschen der Unterlegscheibe 6 verhindern.

In **Fig. 16b** ist eine Unterlegscheibe 6 im Detail dargestellt.

Die Unterlegscheibe 6 weist ein zentrales Loch 33 auf, das sich optional in einen Schlitz 34 fortsetzt, der bis zum Rand der Unterlegscheibe 6 reicht. Der Schlitz 34 ist dabei vorzugsweise etwas schmaler als ein Durchmesser des Schaftunterteils 11, so dass die Unterlegscheibe 6 zum Aufschieben auf das Schaftunterteil 11 leicht (vor-)gespannt werden muss.

Um die Elastizität der Unterlegschiebe 6 zu verbessern, kann, wie in Fig. 16b gezeigt ist, Material aus jenem Anteil der Unterlegscheibe 6 entfernt werden, der auf der dem Schlitz 34 entfernten oder gegenüberliegenden Seite des Loches 33 liegt. Hier ist eine Öffnung gezeigt, die sich an das Loch 33 anschließt oder in dieses übergeht. Wenn das Schaftunterteil 11 beim Einsetzen das zentrale Loch 33 erreicht hat, entspannt sich die Unterlegscheibe 6. So wird verhindert, dass die Unterlegscheibe 6 nach dem vollständigen Einsetzen wieder vom Schaftunterteil 11 abrutscht. Dies kann durch die Verriegelung, Rastung oder Klemmung, die in Fig. 16b zu sehen ist, unterstützt werden.

### Bezugszeichenliste

- 1: Radiusköpfchenimplantat
- 2: Kopf
- 3: Schaft
- 4: Gewindeanker
- 5: Verriegelungselement
- 6: Unterlegscheibe
- 7: Rinne
- 8: Nase
- 9: Durchgangsöffnung des Gewindeankers
- 10: Öffnung des Kopfes
- 11: Schaftunterteil
- 12: Schaftoberteil
- 13: Haltestift
- 14: Bohrungen oder Öffnungen im Schaftoberteil
- 15: Gewinde des Gewindeankers
- 16: Unterbrechungen des Gewindes
- 17: Sägeschablone
- 18: Platte der Sägeschablone
- 19: Verbindungselement der Sägeschablone
- 20: Zunge der Sägeschablone
- 21: Durchgangsöffnung der Sägeschablone
- 22: Knochenbearbeitungswerkzeug
- 23: Raspelelement
- 24: Stiel des Knochenbearbeitungswerkzeugs
- 25: Schaft-Implantationswerkzeug
- 26: elastisches Element des Schaft-Implantationswerkzeugs
- 27: Stifte des Knochenbearbeitungswerkzeugs
- 28: Ratsche
- 29: Kopf-Applikationswerkzeug
- 30: Riegel des Verriegelungselements
- 31: Rastelement des Verriegelungselements
- 32: Schlitz des Verriegelungselements
- 33: Loch der Unterlegscheibe
- 34: Schlitz der Unterlegscheibe
- 35: Umlaufende Nut des Schaftunterteils
- 36: Verliersicherung des Verriegelungselements
- 37: Verliersicherung des Schiebers des Knochenbearbeitungswerkzeugs
- 38: Blattfeder des Knochenbearbeitungswerkzeugs
- 39: Schieber des Knochenbearbeitungswerkzeugs
- 41: Zeiger der Größenlehre
- 42: Skala der Größenlehre
- 43: Größenlehre
- 44: Schlitten der Größenlehre
- 45: Arm der Größenlehre
- 46: Verliersicherung des Raspelelements des Knochenbearbeitungswerkzeugs
- 47: Vertiefung des Raspelelements

## Patentansprüche

1. Radiusköpfchenimplantat (1) mit einem Kopf (2), einem Gewindeanker (4) und einem Schaft (3), wobei der Kopf (2) ein Verriegelungselement (5) zum Befestigen des Kopfes (2) aufweist; wobei der Gewindeanker (4) ein selbstschneidendes Gewinde (15) und eine zentrale Durchgangsöffnung (9) aufweist; und wobei der Schaft (3) ein Schaftoberteil (12) und ein Schaftunterteil (11) aufweist, wobei das Schaftoberteil (12) ausgestaltet ist zur Befestigung an dem Kopf (2) und das Schaftunterteil (11) ausgestaltet ist zum Einführen in einen Radius.

2. Radiusköpfchenimplantat (1) nach Anspruch 1, wobei das Verriegelungselement (5) einen Riegel (30), eine Verliersicherung (36) und/oder ein Rastelement (31) für mindestens eine Rotationsstellung aufweist.

3. Radiusköpfchenimplantat (1) nach einem der vorhergehenden Ansprüche, wobei der Kopf (2) zur Artikulation mit dem Capitulum humeri auf einer Oberseite konkav ausgestaltet ist.

4. Radiusköpfchenimplantat (1) nach einem der vorhergehenden Ansprüche, wobei der Kopf (2) eine Öffnung (10) an einer Unterseite mit mindestens zwei unterschiedlichen Abmessungen in einer Erstreckungsrichtung aufweist.

5. Radiusköpfchenimplantat (1) nach einem der vorhergehenden Ansprüche, wobei der Gewindeanker (4) einen sich nach distal verjüngenden Körper aufweist.

6. Radiusköpfchenimplantat (1) nach einem der vorangegangenen Ansprüche, wobei das Schaftunterteil (11) einführbar ist in die zentrale Durchgangsöffnung (9) des Gewindeankers (4) .

7. Radiusköpfchenimplantat (1) nach einem der vorangegangenen Ansprüche, wobei das Schaftunterteil (11) mindestens eine Rinne (7) aufweist.

8. Radiusköpfchenimplantat (1) nach Anspruch 7, wobei der Gewindeanker (4) mindestens eine Nase (8) aufweist, die in die mindestens eine Rinne (7) des Schaftunterteils (11) eingreifen kann.

9. Radiusköpfchenimplantat (1) nach einem der vorangegangenen Ansprüche, wobei das Schaftoberteil (12) einen Haltestift (13) aufweist zum Zusammenwirken mit dem Kopf (2).

10. Radiusköpfchenimplantat (1) nach einem der vorangegangenen Ansprüche, wobei das Schaftoberteil (12), gemessen von einer Längsachse des Schaftunterteils (11), eine Erstreckung in eine erste Richtung aufweist, die sich unterscheidet von einer Erstreckung in eine zweite Richtung.

11. Radiusköpfchenimplantat (1) nach einem der vorangegangenen Ansprüche, wobei das Schaftoberteil (12) eine Grundfläche aufweist, die von einem Kreisbogen und einer geraden Linie begrenzt ist.

12. Radiusköpfchenimplantat (1) nach einem der vorangegangenen Ansprüche, wobei das Schaftoberteil (12) mindestens eine Bohrung (14) zum Übertragen eines Drehmoments aufweist.

13. Radiusköpfchenimplantat (1) nach einem der vorangegangenen Ansprüche, wobei das Radiusköpfchenimplantat (1) eine Unterlegscheibe (6) zum Einsetzen zwischen Schaft (3) und Knochen aufweist.

14. Kit mit Radiusköpfchenimplantat (1) nach Anspruch 1 bis 13 und mindestens einem der folgenden Hilfsmittel:
- Sägeschablone (17) mit einer Platte (18) und mit einer parallel zur Platte (18) angebrachten Zunge (20) mit einer Konvexität in Richtung der Platte (18);
- Kopf-Applikationswerkzeug (29) zum Aufnehmen des Kopfes (2) und zum Befestigen des Kopfes (2) auf einem in einen Radius eingesetzten Schaft (3);
- Schaft-Implantationswerkzeug (25) zum Einsetzen des Schafts (3) mit Gewindeanker (4) in den Markraum eines Radius;
- Ratsche (28) zum Eindrehen des Schafts (3) samt Gewindeanker (4) in den Radius;
- Knochenbearbeitungswerkzeug (22) mit einem Stiel (24) und einem seitlich angeordneten Raspelelement (23).

## Claims

1. A radial head implant (1) comprising a head (2), a threaded anchor (4) and a shaft (3), wherein the head (2) comprises an interlocking element (5) for fastening the head (2);
wherein the threaded anchor (4) comprises a self-tapping thread (15) and a central through-opening (9);
and wherein the shaft (3) comprises an upper shaft part (12) and a lower shaft part (11), the upper shaft part (12) being designed to be fastened to the head (2) and the lower shaft part (11) being designed to be inserted into a radius.

2. The radial head implant (1) according to claim 1, wherein the interlocking element (5) comprises a latch (30), a securing element (36) and/or a detent element (31) for at least one rotational position.

3. The radial head implant (1) according to anyone of the preceding claims, wherein the head (2) is designed concavely on an upper side for articulation with the capitulum humeri.

4. The radial head implant (1) according to anyone of the preceding claims, wherein the head (2) comprises an opening (10) at a lower side with at least two different dimensions in one extension direction.

5. The radial head implant (1) according to anyone of the preceding claims, wherein the threaded anchor (4) comprises a body which tapers distally.

6. The radial head implant (1) according to anyone of the preceding claims, wherein the lower shaft part (11) can be inserted into the central through-opening (9) of the threaded anchor (4).

7. The radial head implant (1) according to anyone of the preceding claims, wherein the lower shaft part (11) comprises at least one groove (7).

8. The radial head implant (1) according to claim 7, wherein the threaded anchor (4) comprises at least one nose (8) which can engage in the at least one groove (7) of the lower shaft part (11).

9. The radial head implant (1) according to anyone of the preceding claims, wherein the upper shaft part (12) comprises a holding pin (13) for cooperation with the head (2).

10. The radial head implant (1) according to anyone of the preceding claims, wherein the upper shaft part (12), measured from a longitudinal axis of the lower shaft part (11), comprises an extension in a first direction, which differs from an extension in a second direction.

11. The radial head implant (1) according to anyone of the preceding claims, wherein the upper shaft part (12) comprises a base surface which is delimited by a circular arc and a straight line.

12. The radial head implant (1) according to anyone of the preceding claims, wherein the upper shaft part (12) comprises at least one bore (14) for transmitting a torque.

13. The radial head implant (1) according to anyone of the preceding claims, wherein the radial head implant (1) comprises a washer (6) to be inserted between shaft (3) and bone.

14. A kit with a radial head implant (1) according to the claims 1 to 13 and at least one of the following aids:
- sawing template (17) having a plate (18) and a tongue (20) mounted parallel to the plate (18) with a convexity in the direction of the plate (18);
- head-applying tool (29) for receiving the head (2) and for fastening the head (2) on a shaft (3) inserted into a radius;
- shaft-implanting tool (25) for inserting the shaft (3) with threaded anchor (4) into the medullary cavity of a radius;
- ratchet (28) for screwing the shaft (3) together with the threaded anchor (4) into the radius;
- bone processing tool (22) with a stalk (24) and a laterally arranged rasping element (23).

## Revendications

1. Un implant de tête radiale (1) comprenant une tête (2), un ancrage fileté (4) ainsi qu'une tige (3), où la tête (2) comporte un élément de verrouillage (5) pour fixer la tête (2); où l'ancrage fileté (4) comporte un filetage autotaraudant (15) ainsi qu'une ouverture traversante centrale (9) ; et où la tige (3) comporte une partie supérieure de tige (12) ainsi qu'une partie inférieure de tige (11), la partie supérieure de tige (12) étant conçue pour être fixée à la tête (2) et la partie inférieure de tige (11) étant conçue pour être insérée dans un radius.

2. L'implant de tête radiale (1) selon la première revendication, où l'élément de verrouillage (5) comprend un verrou (30), un élément de sécurité anti-perte (36) et/ou un élément d'encliquetage (31) pour au moins une position de rotation.

3. L'implant de tête radiale (1) selon l'une quelconque des revendications précédentes, où la tête (2) est conçue de manière concave sur une face supérieure pour s'articuler avec le capitulum humeri.

4. L'implant de tête radiale (1) selon l'une quelconque des revendications précédentes, où la tête (2) comprend une ouverture (10) sur une face inférieure ayant au moins deux dimensions différentes dans une direction d'extension.

5. L'implant de tête radiale (1) selon l'une quelconque des revendications précédentes, où l'ancrage fileté (4) comporte un corps qui s'effile distalement.

6. L'implant de tête radiale (1) selon l'une quelconque des revendications précédentes, où la partie inférieure de tige (11) peut être insérée dans l'ouverture traversante centrale (9) de l'ancrage fileté (4).

7. L'implant de tête radiale (1) selon l'une quelconque des revendications précédentes, où la partie inférieure de tige (11) comprend au moins une rainure (7).

8. L'implant de tête radiale (1) selon la revendication 7, où l'ancrage fileté (4) comprend au moins un ergot (8) qui peut s'engager dans au moins une rainure (7) de la partie inférieure de tige (11).

9. L'implant de tête radiale (1) selon l'une quelconque des revendications précédentes, où la partie supérieure de tige (12) comporte une goupille de retenue (13) destinée à coopérer avec la tête (2).

10. L'implant de tête radiale (1) selon l'une quelconque des revendications précédentes, où la partie supérieure de tige (12), mesurée depuis un axe longitudinal de la partie inférieure de tige (11), comprend une extension dans une première direction qui diffère d'une extension dans une seconde direction.

11. L'implant de tête radiale (1) selon l'une quelconque des revendications précédentes, où la partie supérieure de tige (12) comporte une surface de base délimitée par un arc de cercle ainsi qu'une ligne droite.

12. L'implant de tête radiale (1) selon l'une quelconque des revendications précédentes, où la partie supérieure de tige (12) comprend au moins un alésage (14) pour transmettre un couple de rotation.

13. L'implant de tête radiale (1) selon l'une quelconque des revendications précédentes, où l'implant de tête radiale (1) comporte une rondelle (6) destinée à être insérée entre la tige (3) et l'os.

14. Un set avec un implant de tête radiale (1) selon les revendications 1 à 13 et au moins un des outils suivants:
- un gabarit de sciage (17) comportant une plaque (18) et une languette (20) montée parallèlement à la plaque (18) avec une convexité en direction de la plaque (18);
- un outil d'application de tête (29) destiné à recevoir une tête (2) et à fixer la tête (2) sur une tige (3) insérée dans un radius;
- un outil d'implantation de tige (25) destiné à insérer la tige (3) avec l'ancrage fileté (4) dans la cavité médullaire d'un radius;
- un cliquet (28) destiné à visser la tige (3) avec l'ancrage fileté (4) dans le radius;
- un outil de traitement d'os (22) comportant un manche (24) ainsi qu'une râpe (23) agencée latéralement.
